# EUROPÄISCHE PATENTSCHRIFT

(11) **EP 0 496 701 B1**
(45) Veröffentlichungstag und Bekanntmachung des Hinweises auf die Patenterteilung: **06.03.1996**
(21) Anmeldenummer: 92810027.0
(22) Anmeldetag: 16.01.1992
(51) Int. Cl.: C07D 409/12, A01N 47/36, C07D 405/12, C07D 331/04, C07D 305/08

(54) **Sulfonylharnstoffe als Herbizide**
Sulfonylureas as herbizides
Sulfonylurées comme herbicides

(30) Priorität: 25.01.1991 CH 220/91
(43) Veröffentlichungstag der Anmeldung: 29.07.1992
(73) Patentinhaber: CIBA-GEIGY AG, CH-4002 Basel (CH)
(72) Erfinder: Meyer, Willy, CH-4125 Riehen (CH)

(56) Entgegenhaltungen:
- EP-A- 0 007 687
- EP-A- 0 030 138
- EP-A- 0 291 851
- US-A- 4 892 946

## Beschreibung

### Neue Sulfonylharnstoffe

Die vorliegende Erfindung betrifft neue, herbizid wirksame und pflanzenwuchsregulierende N-Phenylsulfonyl-N'-pyrimidinyl-, N'-triazinyl- und N'-triazolylharnstoffe und -thioharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Harnstoffverbindungen, Triazinverbindungen und Pyrimidinverbindungen mit herbizider Wirkung sind allgemein bekannt. Derartige Verbindungen werden beispielsweise in den Europäischen Patentanmeldungen Nr. 0 007 687, 0 030 138, 0 073 562 und 0 126 711 beschrieben.

Es wurden nun neue Sulfonylharnstoffe und -thioharnstoffe mit herbiziden und pflanzenwachstumsregulierenden Eigenschaften gefunden.

Die erfindungsgemäßen N-Phenylsulfonyl-N'-pyrimidinyl-, N'-triazinyl- und N'-triazolylharnstoffe und -thioharnstoffe entsprechen der Formel I 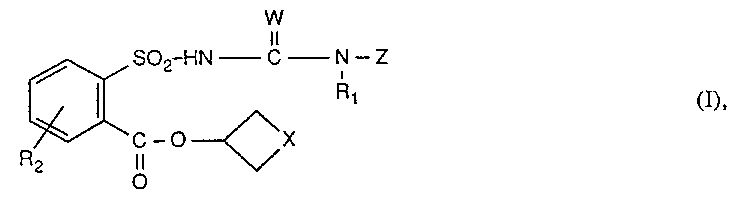 worin
- X: für Sauerstoff,
- W: für Sauerstoff oder Schwefel;
- R₁: für Wasserstoff oder Methyl;
- R₂: für Wasserstoff, Fluor, Chlor, Brom, Jod, (X)ₙR₃, NO₂, NR₄R_{S}, -C≡CR₆, 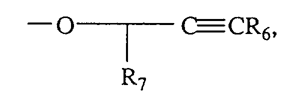 oder Cyano;
- n: für die Zahl 0 oder 1;
- R₃: für C₁-C₄-Alkyl, oder durch 1-4 Halogenatome, C₁-C₃-Alkoxy oder C₁-C₃-Alkylthio substituiertes C₁-C₄-Alkyl; C₂-C₄Alkenyl, oder durch 1-4 Halogenatome substituiertes C₂-C₄Alkenyl;
- R₄: für Wasserstoff, CH₃O, CH₃CH₂O oder C₁-C₃-Alkyl;
- R₅: für Wasserstoff oder C₁-C₃-Alkyl;
- R₆: für Wasserstoff, Methyl oder Ethyl;
- R₇: für Wasserstoff oder Methyl;
- Z: für 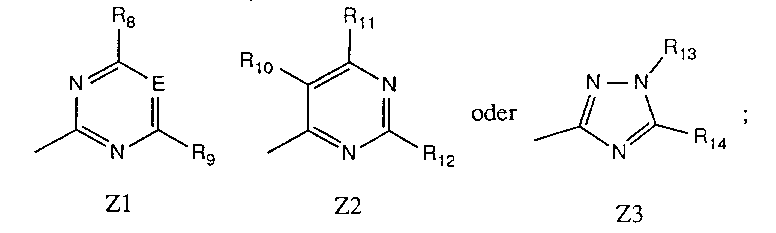
- E: für Methin oder Stickstoff;
- R₈: für C₁-C₄-Alkyl, C₁-C₄-Alkoxy, C₁-C₄-Halogenalkoxy, C₁-C₄-Halogenalkyl, C₁-C₄-Halogenalkylthio, C₁-C₄-Alky-thio, Halogen, C₂-C₅-Alkoxyalkyl, C₂-C₅-Alkoxyalkoxy, Amino, C₁-C₃-Alkylamino oder Di-(C₁-C₃Alkyl)amino;
- R₉: für C₁-C₄-Alkyl, C₁-C₄-Alkoxy, C₁-C₄-Halogenalkoxy, C₁-C₄-Halogenalkylthio, C₁-C₄-Alkylthio, C₂-C₅-Alkoxyalkyl, C₂-C₅-Alkoxyalkoxy, C₂-C₅-Alkylthioalkyl oder Cyclopropyl;
- R₁₀: für Wasserstoff, Fluor, Chlor, Methyl, Trifluormethyl, CH₃O, CH₃CH₂O, CH₃S, CH₃SO, CH₃SO₂ oder Cyano;
- R₁₁: für Methyl, Ethyl, CH₃O, CH₃CH₂O, Fluor oder Chlor;
- R₁₂: für Methyl, Ethyl, CH₃O, CH₃CH₂O, Fluor oder Chlor;
- R₁₃: für C₁-C₃-Alkyl;
- R₁₄: für C₁-C₃-Alkyl, C₁-C₃-Alkoxy, Chlor oder OCHF₂ stehen;
sowie den Salzen dieser Verbindungen;
mit den Maßgaben, daß
E für Methin steht wenn R₈ Halogen bedeutet; und
E für Methin steht wenn R₈ oder R₉ OCHF₂ oder SCHF₂ bedeuten.

In den obigen Definitionen ist unter Halogen, Fluor, Chlor, Brom und Jod, vorzugsweise Fluor, Chlor und Brom zu verstehen.

Die in den Substituentendefinitionen vorkommenden Alkylgruppen können geradkettig oder verzweigt sein und stehen beispielsweise für Methyl, Ethyl, n-Propyl, iso-Propyl, n-Butyl, sek.-Butyl, iso-Butyl oder tert.-Butyl. Vorzugsweise besitzen die als oder in den Substituenten vorkommendenen Alkylgruppen 1-3 Kohlenstoffatome.

Unter Alkenyl ist geradkettiges oder verzweigtes Alkenyl zu verstehen, wie z.B. Vinyl, Allyl, Methallyl, 1-Methylvinyl oder But-2-en-1-yl. Bevorzugt sind Alkenylreste mit einer Kettenlänge von 2 bis 3 Kohlenstoffatomen.

Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl, Trifluormethyl und Dichlorfluormethyl.

Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.

Halogenalkoxy ist z.B. Difluormethoxy, Trifluormethoxy, 2,2,2-Trifluorethoxy, 1,1,2,2-Tetrafluorethoxy, 2-Fluorethoxy, 2-Chlorethoxy und 2,2-Difluorethoxy; vorzugsweise Difluormethoxy, 2-Chlorethoxy und Trifluormethoxy.

Alkylthio ist beispielsweise Methylthio, Ethylthio, Propylthio, Isopropylthio, n-Butylthio, iso-Butylthio, sek.-Butylthio oder tert.-Butylthio, vorzugsweise Methylthio und Ethylthio.

Beispiele für Alkoxyalkoxy sind: Methoxymethoxy, Methoxyethoxy, Methoxypropyloxy, Ethoxymethoxy, Ethoxyethoxy sowie Propyloxymethoxy.

Alkylamino ist beispielsweise Methylamino, Ethylamino, n-Propylamino oder iso-Propylamino. Dialkylamino steht beispielsweise für Dimethylamino, Methylethylamino, Diethylamino oder n-Propylmethylamino.

Die Erfindung umfaßt ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, iso-Propylamin, die vier isomeren Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und iso-Chinolin, insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Teaamethylammoniumkation, das Trimethylbenzylammoniumkation, das Triethylbenzylammoniumkation, das Tetraethylammoniumkation, das Trimethylethylammoniumkation, aber auch das Ammoniumkation.

Unter den Verbindungen der Formel I sind diejenigen bevorzugt, in denen W für Sauerstoff steht, wobei vorzugsweise Z für Z1 und X für Sauerstoff steht und E Stickstoff bedeutet.

Ferner ist diejenige Gruppe von Verbindungen der Formel I hervorzuheben, in der Z für Z1 und X für Sauerstoff steht und E Methin bedeutet.

Aus diesen beiden Gruppen von Verbindungen der Formel I sind diejenigen von besonderem Interesse, in denen
R₂ für Wasserstoff, Fluor, Chlor, OCH₃, OCHF₂, Methyl, SCH₃, Methoxy, Ethoxy oder Chlorethoxy;
R₈ für C₁-C₃-Alkyl, C₁-C₃-Alkoxy, C₁-C₂-Halogenalkoxy, Trifluormethyl, CHF₂, CH₂F, CH₂OCH₃, Fluor, Chlor, NH₂, NHCH₃, N(CH₃)₂, SCH₃ oder CH₂OCH₃; und
R₉ für C₁-C₃-Alkyl, C₁-C₃-Alkoxy, C₁-C₂Halogenalkoxy oder Cyclopropyl stehen.

Bei besonders bevorzugten Verbindungen aus dieser Gruppe stehen
R₂ für Wasserstoff;
R₈ für Methyl, Ethyl, OCH₃, OC₂H₅, OCHF₂, OCH₂CF₃, Chlor, NHCH₃, N(CH₃)₂ oder CH₂OCH₃; und
R₉ für Methyl, OCH₃, OCHF₂, OC₂H₅ oder Cyclopropyl.

Als bevorzugte Einzelverbindungen aus dem Umfang der Formel I sind zu nennen:
N-[2-(Oxetan-Oxy-carbonyl)]phenylsulfonyl-N'-(4-methoxy-6-methyl-1,3,5-triazinyl)-harnstoff;
N-[2-(Oxetan-3-oxy-carbonyl)]-phenylsulfonyl-N'-(4,6-dimethyl-pyrimidin-2-yl)-harnstoff;
N-[2-(Oxetan-3-oxy-carbonyl)]-phenylsulfonyl-N'-(4-methoxy-6-methyl-pyrimidin-2-yl)-harnstoff; und
N-[2-(Oxetan-3-oxy-carbonyl)]-phenylsulfonyl-N'-(4,6-dimethoxy-pyrimidin-2-yl)-harnstoff.

Die Verbindungen der Formel I können hergestellt werden, indem man entweder
a) ein Phenylsulfonamid der Formel II 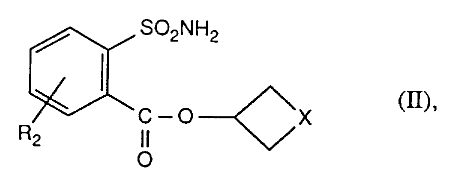 worin R₂ und X die unter Formel I angegebene Bedeutung haben, in Gegenwart einer Base mit einem Pyrimidinyl-, Triazolyl- oder Triazinylcarbamat oder -thiocarbamat der Formel III 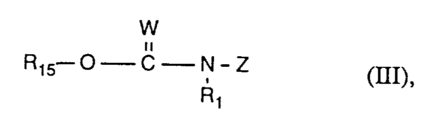 worin W, Z und R₁ die unter Formel I angegebene Bedeutung haben und R₁₅ für Phenyl, oder substituiertes Phenyl steht, umsetzt, oder
b) ein Sulfonylcarbamat oder -thiocarbamat der Formel IV 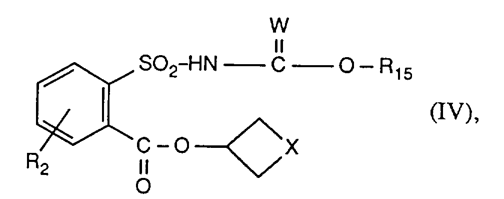 worin R₂, W, X und Z die unter Formel I angegebene Bedeutung haben und R₁₅ die unter Formel III angegebene Bedeutung hat, in Gegenwart einer Base mit einem Amin der Formel V

   H₂N-Z (V)

   worin Z die unter Formel I angegebene Bedeutung hat, umsetzt, oder
c) ein Phenylsulfonamid der Formel II 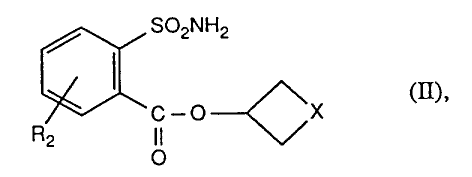 worin R₂ und X die unter Formel I angegebene Bedeutung haben, in Gegenwart einer Base mit einem Pyrimidinyl-, Triazolyl- oder Triazinylisocyanat oder -isothiocyanat der Formel VII

   Y=N=C-Z (VII)

   worin Z die unter Formel I angegebene Bedeutung hat und Y Sauerstoff oder Schwefel bedeutet, umsetzt.

Verbindungen der Formel I können ferner hergestellt werden indem man eine Verbindung der Formel VIII mit einer Verbindung der Formel V in Gegenwart eines Ammonium-, Phosphonium-, Sulfonium- oder Alkalimetallcyanatsalzes der Formel X

M⁺OCN⁻ (X)

worin M für ein Alkalimetall oder für die Gruppe R₁₅ R₁₆ R₁₇ R₁₈Q steht, worin R₁₅ R₁₆ R₁₇ R₁₈ unabhängig voneinander C₁-C₁₈-Alkyl, Benzyl oder Phenyl bedeutet, wobei die Gesamtzahl der C-Atome nicht größer als 36 ist; und Q Stickstoff, Schwefel oder Phosphor bedeutet, umsetzt. Nach dieser Methode lassen sich besonders vorteilhaft die Verbindungen N-[2-(Oxetan-3-oxy-carbonyl)]-phenylsulfonyl-N′-(4-methoxy-6-methyl-1,3,5-triazinyl)-harnstoff;
N- [2-(Oxetan-3-oxy-carbonyl)]-phenylsulfonyl-N′-(4,6-dimethyl-pyrimidin-2-yl)-harnstoff;
N- [2-(Oxetan-3-oxy-carbonyl)]-phenylsulfonyl-N′-(4-methoxy-6-methyl-pyrimidin-2-yl)-harnstoff; und
N- [2-(Oxetan-3-oxy-carbonyl)]-phenylsulfonyl-N′-(4,6-dimethoxy-pyrimidin-2-yl)-harnstoff herstellen. Derartige Umsetzungen werden in der schweizerischen Patentschrift Nr. 662 348 beschrieben.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C.
Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffe oder chlorierten Kohlenwasserstoffe, gereinigt werden.

In den oben beschriebenen Herstellungsverfahren der Verbindungen der Formel I steht R₁₅ vorzugsweise für Phenyl, welches durch C₁-C₄-Alkyl oder Halogen substituiert sein kann, ganz besonders bevorzugt für Phenyl.

Die Phenylsulfonamide der Formel II sind neue Verbindungen die speziell für die Herstellung der Wirkstoffe der Formel I entwickelt und hergestellt wurden. Sie bilden daher einen Gegenstand der vorliegenden Erfindung. Sie lassen sich aus den entsprechenden Phenylsulfochloriden der Formel VIII 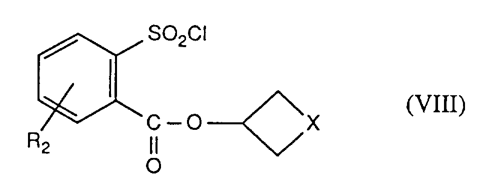 worin R₂ und X die unter Formel I angegebene Bedeutung haben, durch Umsetzung mit Ammoniak herstellen. Derartige Umsetzungen sind bekannt und dem Fachmann geläufig.

Die Phenylsulfochloride der Formel VIII sind neue Verbindungen die speziell für die Herstellung der Wirkstoffe der Formel I entwickelt und hergestellt wurden. Sie bilden daher ebenfalls einen Gegenstand der vorliegenden Erfindung. Die Phenylsulfochloride der Formel VIII werden hergestellt, indem man die entsprechend substituierten 2-Chlorsulfonyl-benzoylchloride (siehe z.B. D.Davis, Soc. 2042, 2044 (1932)) in Gegenwart einer Base mit einer Verbindung der Formel IX 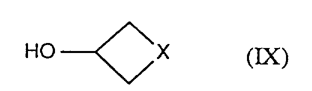 worin X die unter Formel I angegebene Bedeutung hat, umsetzt. Derartige Reaktionen sind bekannt und dem Fachmann geläufig.

Phenylsulfochloride der Formel VIII, worin X für Sauerstoff steht, können auch hergestellt werden, indem man 2-Isopropylthiobenzoesäure (siehe z.B. H.Gilman, F.J.Webb, Am. Soc 71, 4062-4063) mit Thionylchlorid zum entsprechenden Benzoesäurechlorid umsetzt, welches anschließend mit 3-Hydroxy-oxetan in Gegenwart einer Base zum entsprechenden 2-Isopropylthio-benzoesäureoxetan-3-ylester überführt wird, um schließlich durch Umsetzung mit Chlor das Sulfochlorid der Formel VIII zu erhalten. Derartige Umsetzungen sind bekannt und dem Fachmann geläufig.

Verbindungen der Formel IX und deren Herstellung sind bekannt (siehe z.B. B.Lamm et al., Acta Chem. Scand. 28, 701 (1974) oder J. Org. Chem. 48, 2953-2956 (1983)).

Die Sulfonylcarbamate und -thiocarbamate der Formel IV sind neu und bilden einen Gegenstand dervorliegenden Erfindung. Sie können beispielsweise durch Umsetzung der Sulfonamide der Formel II mit Diphenylcarbamat bzw. -thiocarbamat in Gegenwart einer Base erhalten werden. Derartige Reaktionen sind bekannt und dem Fachmann geläufig.

Die Amine der Formel V werden in den europäischen Patentanmeldungen Nr. 0 007 687, 0 030 138, 0 073 562 und 0 126 711 sowie im USP 4,579,584 beschrieben.

Verfahren zur Herstellung von N-Pyrimidinyl- und N-Triazinylcarbamaten sind beispielsweise in EP-A-0101670 beschrieben. N-Triazolylcarbamate können analog hergestellt werden.

Die Wirkstoffe der Formel I werden in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindungen der Formel I zeichnen sich durch wuchshemmende und herbizide Eigenschaften aus, die sie ausgezeichnet zum Einsatz in Kulturen von Nutzpflanzen, insbesondere in Geaeide, Baumwolle, Soja, Raps, Mais und Reis befähigen, wobei der Einsatz in Sojakultur und Getreide ganz besonders bevorzugt ist. Vorzugsweise erfolgt die Bekämpfung der Unkräuter in Sojakulturen postemergent. Insbesondere zeichnen sich die Verbindungen der Formel I durch ihre gute Abbaubarkeit aus.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindungen der Formel I oder sie enthaltender Mittel zur Regulierung des Pflanzenwachstums kommen verschiedene Methoden und Techniken in Betracht, wie beispielsweise die folgenden:

### i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.
b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).
c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Ziekultur gerichtet ist. Man verwendet in der Regel 0,01 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

### ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen C₈ bis C₁₂, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser, Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren (C₁₀-C₂₂), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:
- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

### Emulgierbare Konzentrate:

| | |
|---|---|
| Aktiver Wirkstoff: | 1 bis 20%, vorzugsweise 5 bis 10 % |
| oberflächenaktives Mittel: | 5 bis 30%, vorzugsweise 10 bis 20 % |
| flüssiges Trägermittel: | 15 bis 94%, vorzugsweise 70 bis 85% |

### Stäube:

| | |
|---|---|
| Aktiver Wirkstoff: | 0,1 bis 10%, vorzugsweise 0,1 bis 1 % |
| festes Trägermittel: | 99,9 bis 90%, vorzugsweise 99,9 bis 99 % |

### Suspensions-Konzentrate:

| | |
|---|---|
| Aktiver Wirkstoff: | 5 bis 75%, vorzugsweise 10 bis 50 % |
| Wasser: | 94 bis 24%, vorzugsweise 88 bis 30 % |
| oberflächenaktives Mittel: | 1 bis 40%, vorzugsweise 2 bis 30 % |

### Benetzbare Pulver:

| | |
|---|---|
| Aktiver Wirkstoff: | 0,5 bis 90%, vorzugsweise 1 bis 80 % |
| oberflächenaktives Mittel: | 0,5 bis 20%, vorzugsweise 1 bis 15 % |
| festes Trägermaterial: | 5 bis 95 %, vorzugsweise 15 bis 90 % |

### Granulate:

| | |
|---|---|
| Aktiver Wirkstoff: | 0,5 bis 30%, vorzugsweise 3 bis 15 % |
| festes Trägermittel: | 99,5 bis 70 %, vorzugsweise 97 bis 85 % |

### Herstellungsbeispiele:

### Beispiel H4:

### 2-Isopropylthio-benzoesäurechlorid:

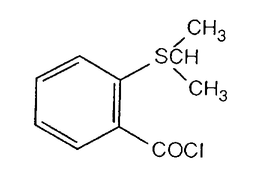

Eine Mischung von 15,7 g 2-Isopropylthio-benzoesäure und 15,7 g Thionylchlorid wird sehr langsam zum Rückfluß erwärmt und bis zur Beendigung der Gasentwicklung unter Rückfluß gehalten. Durch vollständiges Abdampfen des überschüssigen Thionylchlorides erhält man 17,3 g ungereinigtes 2-Isopropylsulfonyl-benzoesäurechlorid als gelbes Öl.

### Beispiel H5:

### 2-Isopropylthio-benzoesäureoxetan-3-ylester:

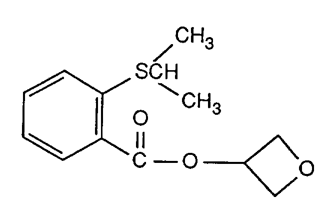

Eine Mischung von 4,44 g 3-Hydroxy-oxetan, 6,64 g Pyridin und 60 ml absolutem Toluol werden bei einer Temperatur von 15 bis 20°C tropfenweise mit einer Mischung von 12,9 g 2-Isopropylthio-benzoesäurechlorid und 20 ml absolutem Toluol versetzt. Die entstandene Suspension wird 2 Stunden bei einer Temperatur von 20-25°C und weitere 3 Stunden bei einer Temperatur von 40-45°C gerührt. Durch Versetzen des Reaktionsgemisches mit Wasser, Waschen der organischen Phase mit Wasser, Trocknen und Eindampfen erhält man 12,6 g 2-Isopropylthio-benzoesäureoxetan-3-ylester als hellgelbes Öl.

### Beispiel H6:

### 2-(Oxetan-3-oxycarbonyl)-phenylsulfochlorid:

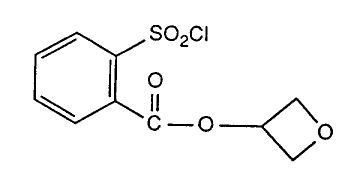

In eine Mischung von 12,6 g 2-Isopropylthio-benzoesäureoxetan-3-ylester, 12,9 g Natriumacetat und 100 ml 50 %ige Essigsäure wird bei einer Temperatur von - 5° bis 0°C während 1 Stunde 11,2 g Chlor eingeleitet und anschließend 15 Minuten bei einer Temperatur von 0°C gerührt. Durch Versetzen des Reaktionsgemisches mit Methylenchlorid, Waschen der organischen Phase mit Eiswasser und Trocknen erhält man eine Methylenchloridlösung von 2-(Oxetan-3-oxycarbonyl)-phenylsulfochlorid, welches ohne weitere Reinigung in Beispiel H7 eingesetzt wird.

### Beispiel H7:

### 2-(Oxetan-3-oxycarbonyl)-phenylsulfonamid:

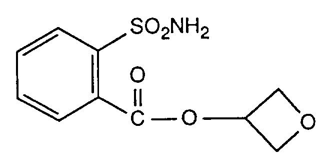

In eine Methylenchloridlösung von 2-(Oxetan-3-oxycarbonyl)-phenylsulfochlorid (Beispiel H6) wird während 45 Minuten bei einer Temperatur von 0° bis 5°C 2,5 g Ammoniak eingeleitet. Durch Versetzen des Reaktionsgemisches mit Wasser, Waschen der organischen Phase mit Wasser, Trocknen, Eindampfen und Kristallisieren aus einem Methylenchlorid/Diethylether-Gemisch erhält man 6,7 g 2-(Oxetan-3-oxycarbonyl)-phenylsulfonamid mit einem Schmelzpunkt von 169 bis 170°C.

### Beispiel H8:

### N-[2-(Oxetan-3-oxycarbonyl)-phenylsulfonyl]-N′-(4-methoxy-6-methyl-1,3,5-triazinyl)-harnstoff (Verbindung Nr. 3.011):

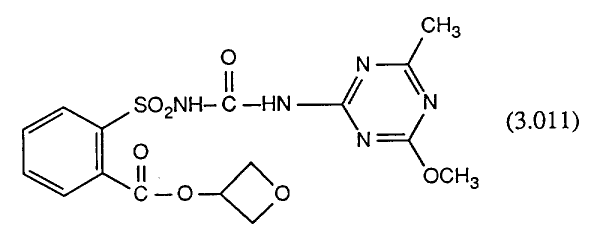

Eine Mischung von 2,57 g 2-(Oxetan-3-oxycarbonyl)-phenylsulfonamid, 2,6 g 4-Methoxy-6-methyl-1,3,5-triazinyl-phenylcarbamat und 40 ml absolutem Dioxan werden bei 20-25°C tropfenweise mit einer Mischung von 1,52 g Diazabicyclo-[5.4.0]-undec-7-en(1.5-5) und 5 ml absolutem Dioxan versetzt und anschließend 4 Stunden bei einer Temperatur von 20 bis 25°C gerührt. Durch Eingeben in Wasser, Zutropfen von 10 %iger Salzsäure bis zur Einstellung eines pH von 5, Extrahieren mit Essigester, Trocknen der organischen Phase, Eindampfen und Kristallisieren aus Essigsäureethylester erhält man 2,8 g N- [2-(Oxetan-3-oxycarbonyl)-phenylsulfonyl]-N'-(4-methoxy-6 -methyl-1,3,5-triazinyl)-harnstoff (Verbindung Nr. 3.011) mit einem Schmelzpunkt von 162 bis 163°C (Zers.).

### Beispiel H9: N-[2-(Oxetan-3-oxycarbonyl)-phenylsulfonyl]-phenylcarbamat (Verbindung Nr. 1.030):

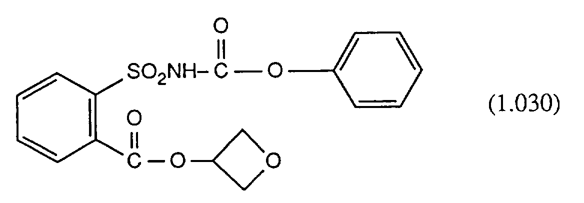

Eine Mischung aus 2,57 g 2-(Oxetan-3-oxycarbonyl)-phenylsulfonamid, 2,14 g Diphenylcarbonat, 1,38 g Kaliumcarbonat und 13 ml Dimethylformamid läßt man bei einer Temperatur von 20-25°C 15 Stunden lang rühren. Anschließend gibt man die Reaktionsmischung in Eiswasser, stellt den pH-Wert durch Zutropfen 10%iger Salzsäure auf den Wert 5-6 ein, extrahiert mit Essigsäureethylester und wäscht mit Wasser. Nach Trocknen über Natriumsulfat, Eindampfen und Kristallisieren des Rückstandes aus Diethylether erhält man 2,2 g N-[2-(Oxetan-3-oxycarbonyl)phenylsulfonyl]-phenylcarbamat (Verbindung 1.030) mit einem Schmelzpunkt von 91-92°C.

### Beispiel H10: N-[2-(Oxetan-3-oxcarbonyl)-phenylsulfonyl]-N'[-4-methoxy-6-(2,2,2-trifluorethoxy-)pyrimidin-2-yl]-harnstoff(Verbindung Nr. 2.034):

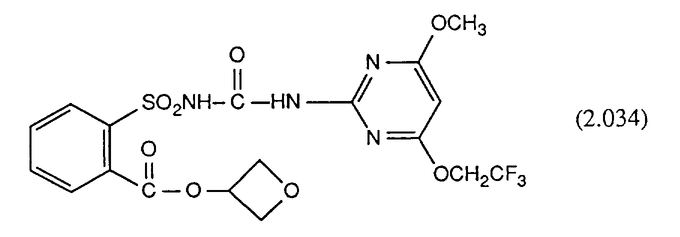

Eine Mischung aus 0,75 g N-[2-(Oxetan-3-oxycarbonyl)-phenylsulfonyl]-phenylcarbamat, 0,34 g 2-Amino-4-methoxy-6-(2,2,2-trifluorethoxy)-pyrimidin und 7 ml Dioxan läßt man bei einer Temperatur von 90-95°C 4 Stunden lang rühren. Nach Eindampfen der Reaktionsmischung und Kristallisieren des Rückstandes aus Aceton erhält man 0,7 g N-[2-(Oxetan-3-oxycarbonyl)-phenylsulfonyl]-N′-[4-methoxy-6-(2,2,2-trifluorethoxy-)pyrimidin-2-yl]-harnstoff (Verbindung Nr. 2.034) mit einem Schmelzpunkt von 185-186°C.

In analoger Weise werden die in den angeschlossenen Tabellen aufgelisteten Verbindungen der Formel I sowie deren Zwischenprodukte hergestellt.

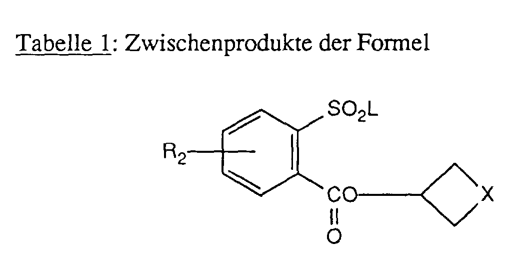

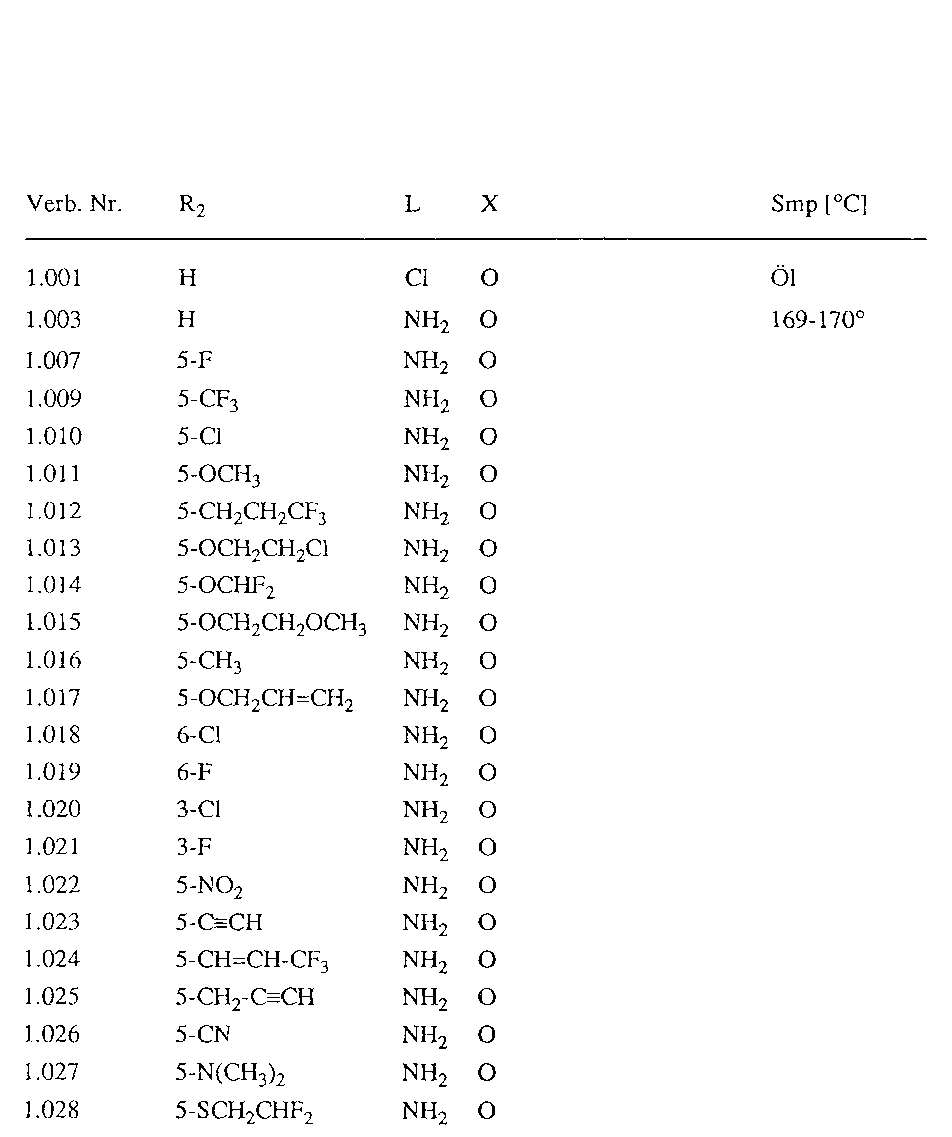

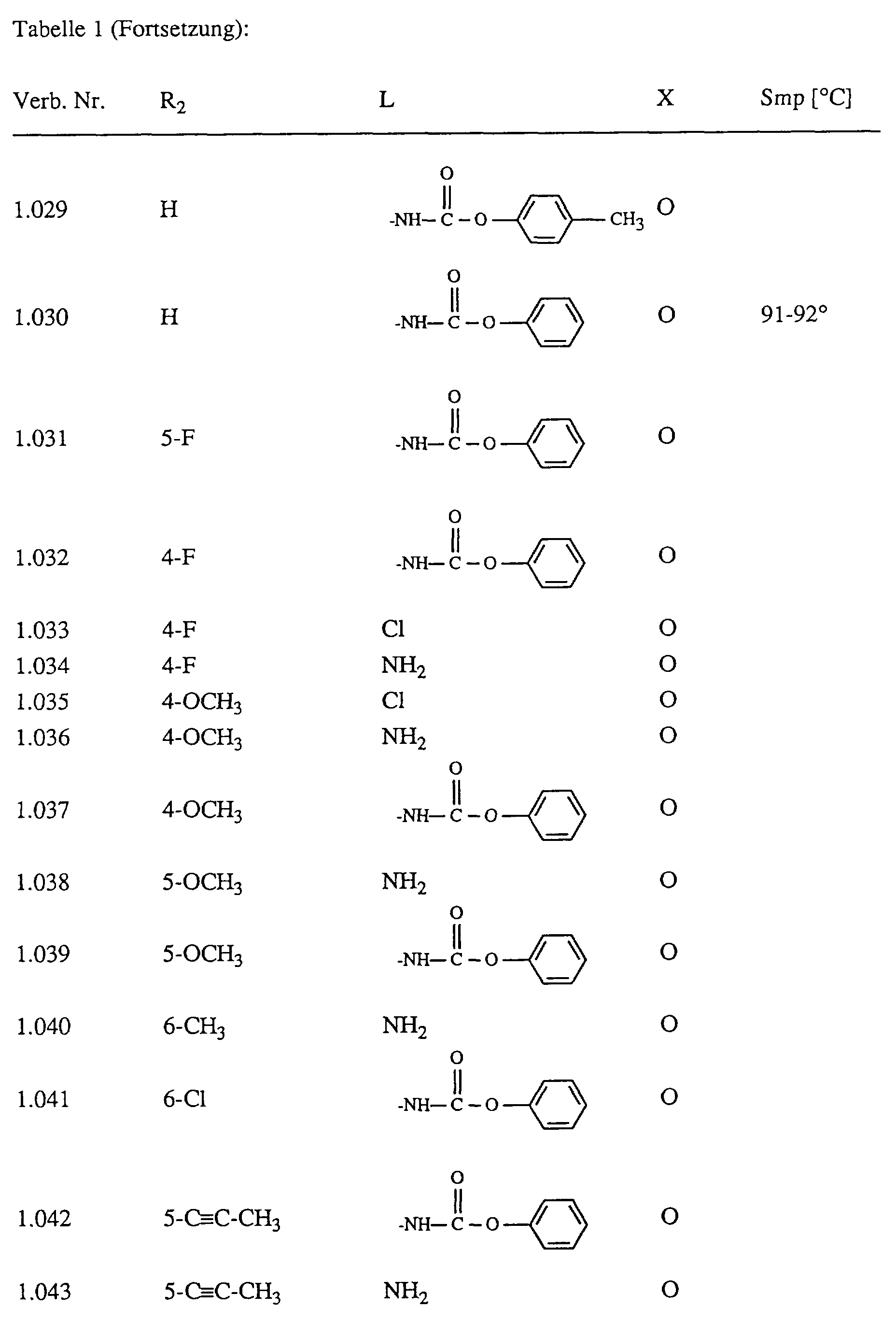

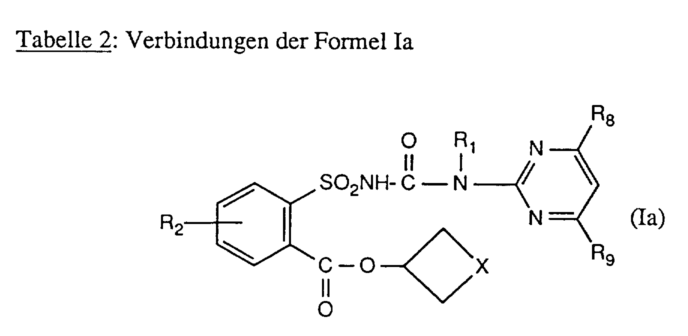

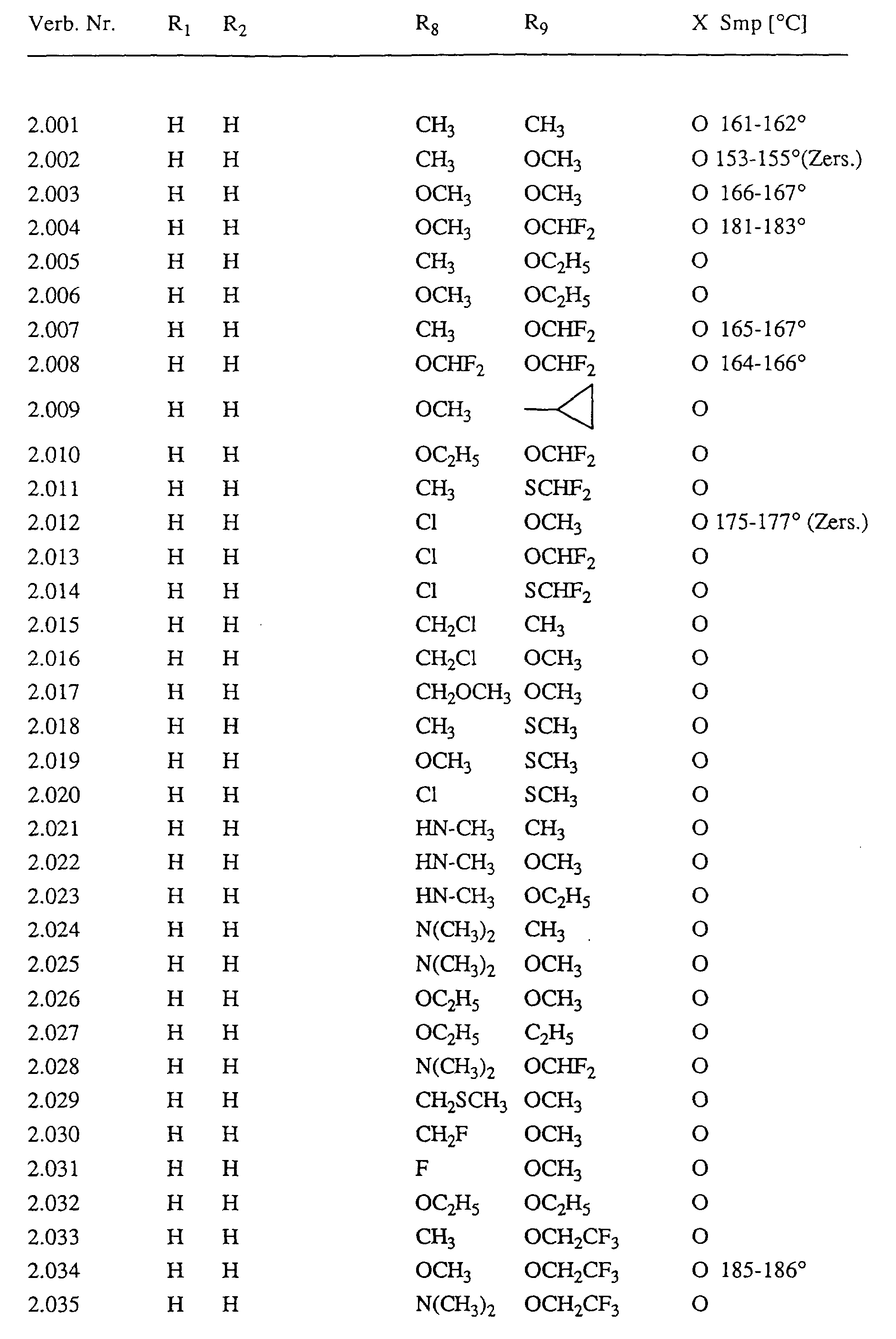

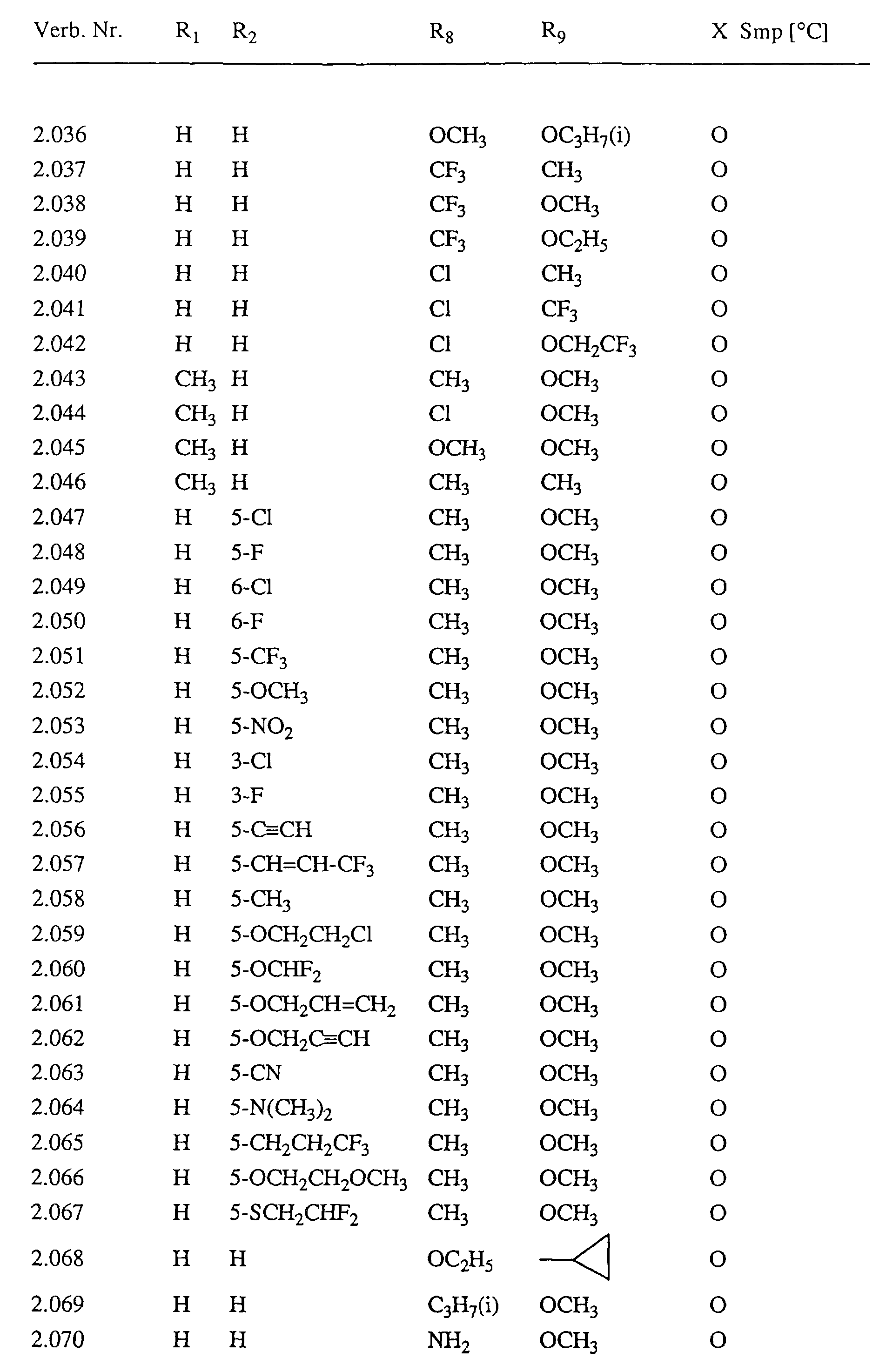

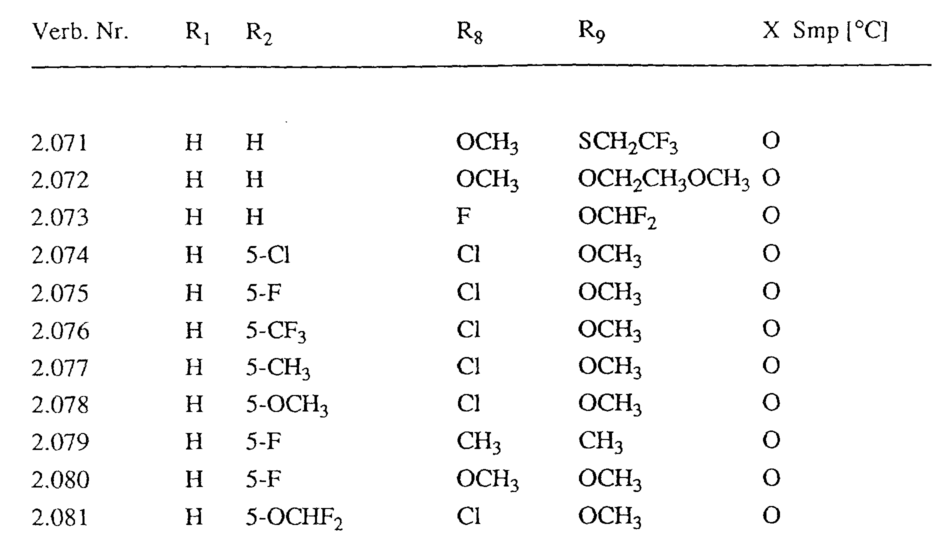

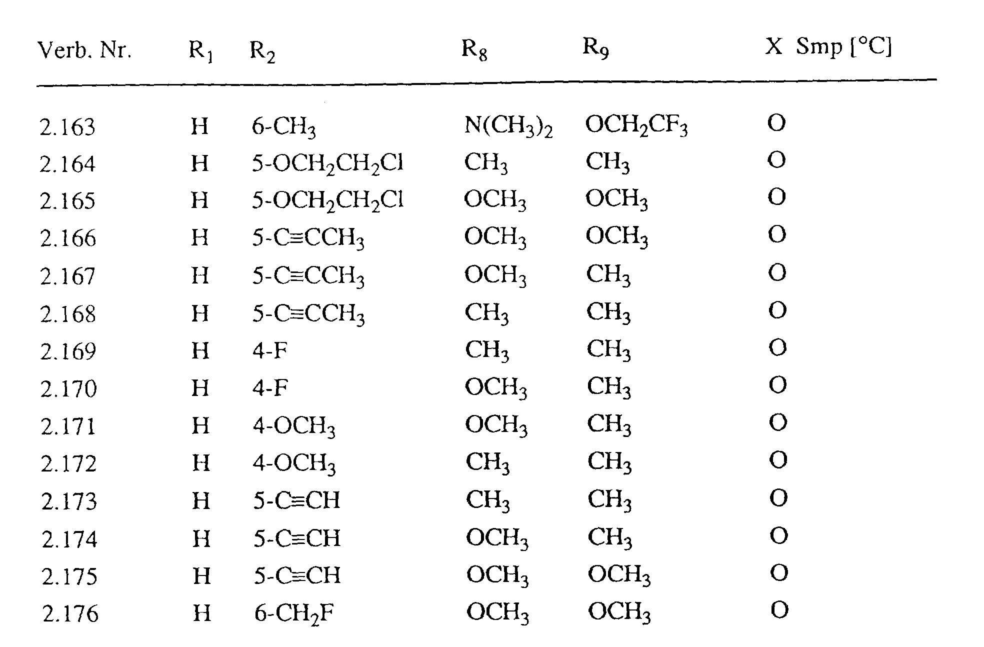

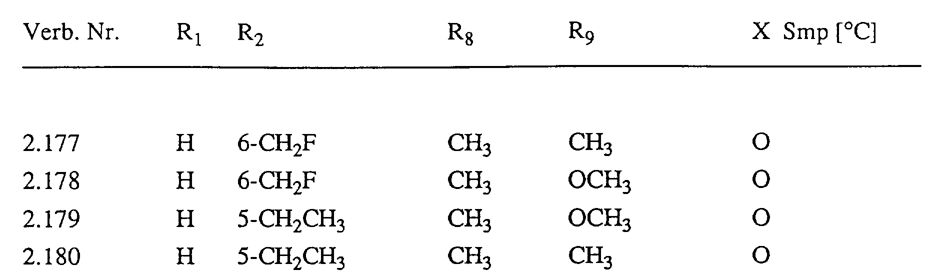

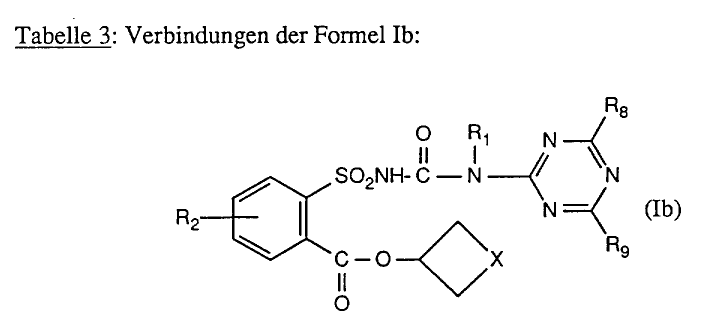

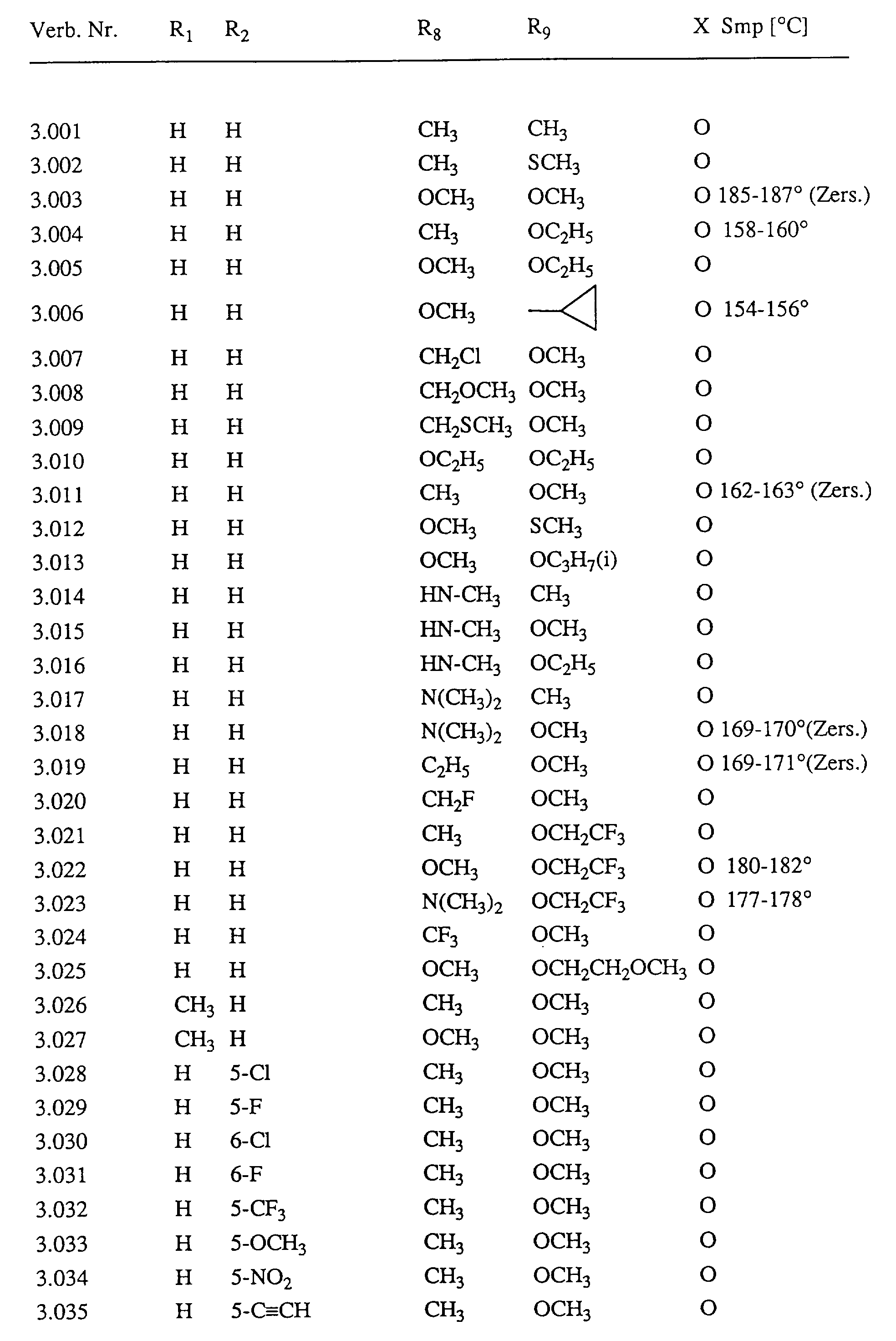

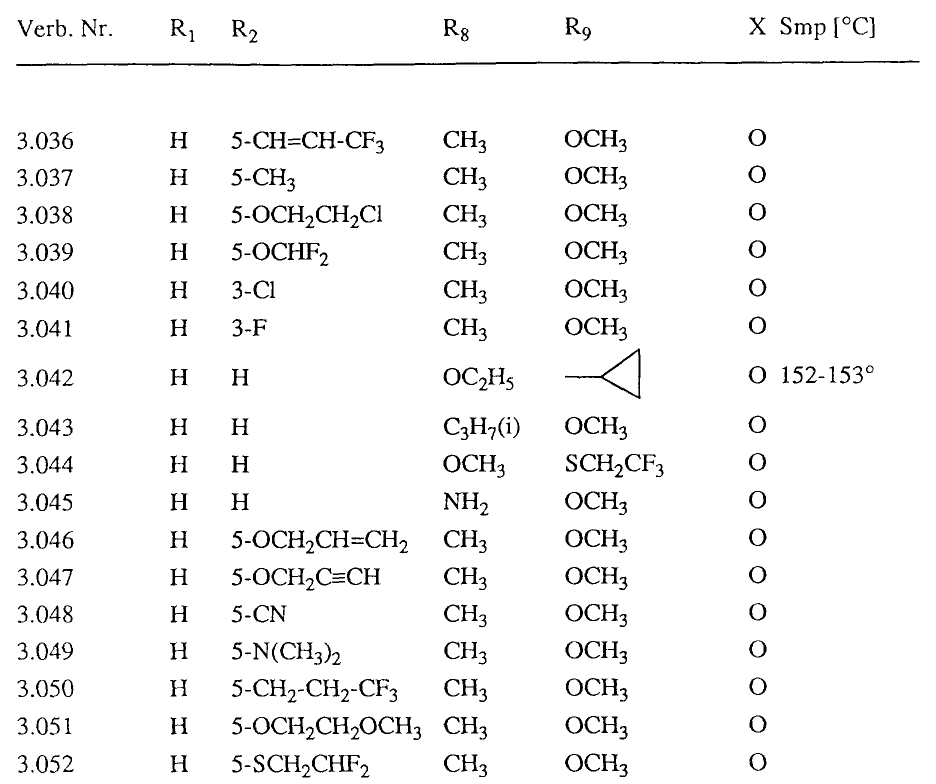

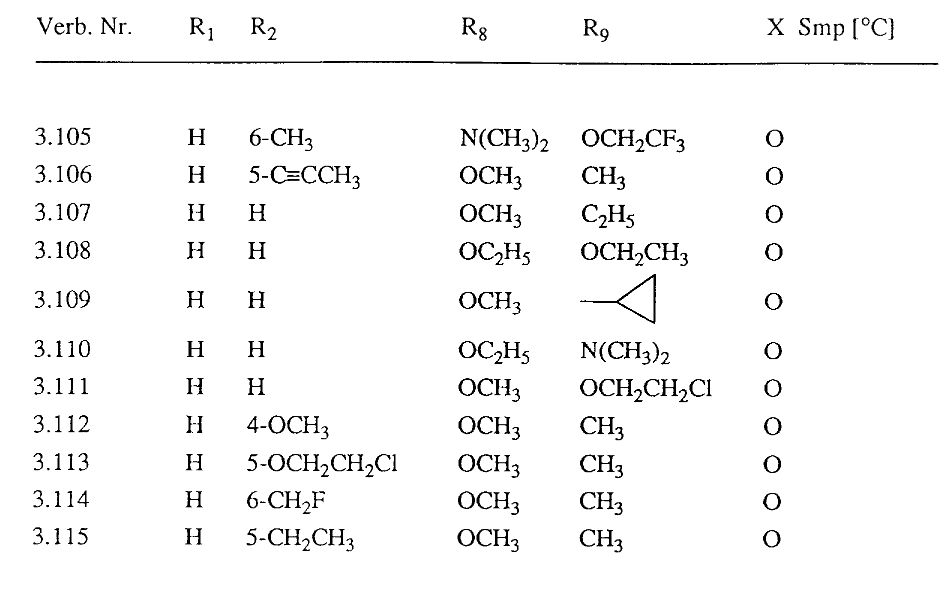

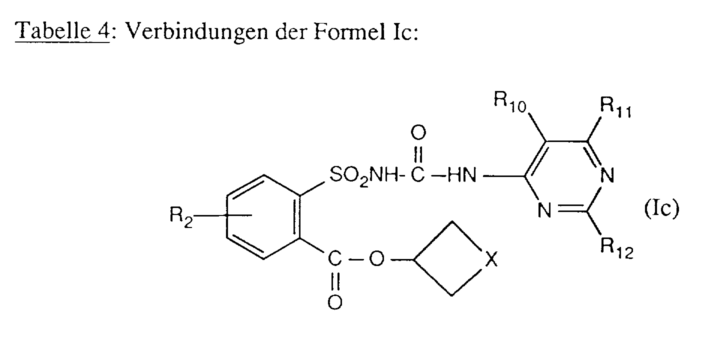

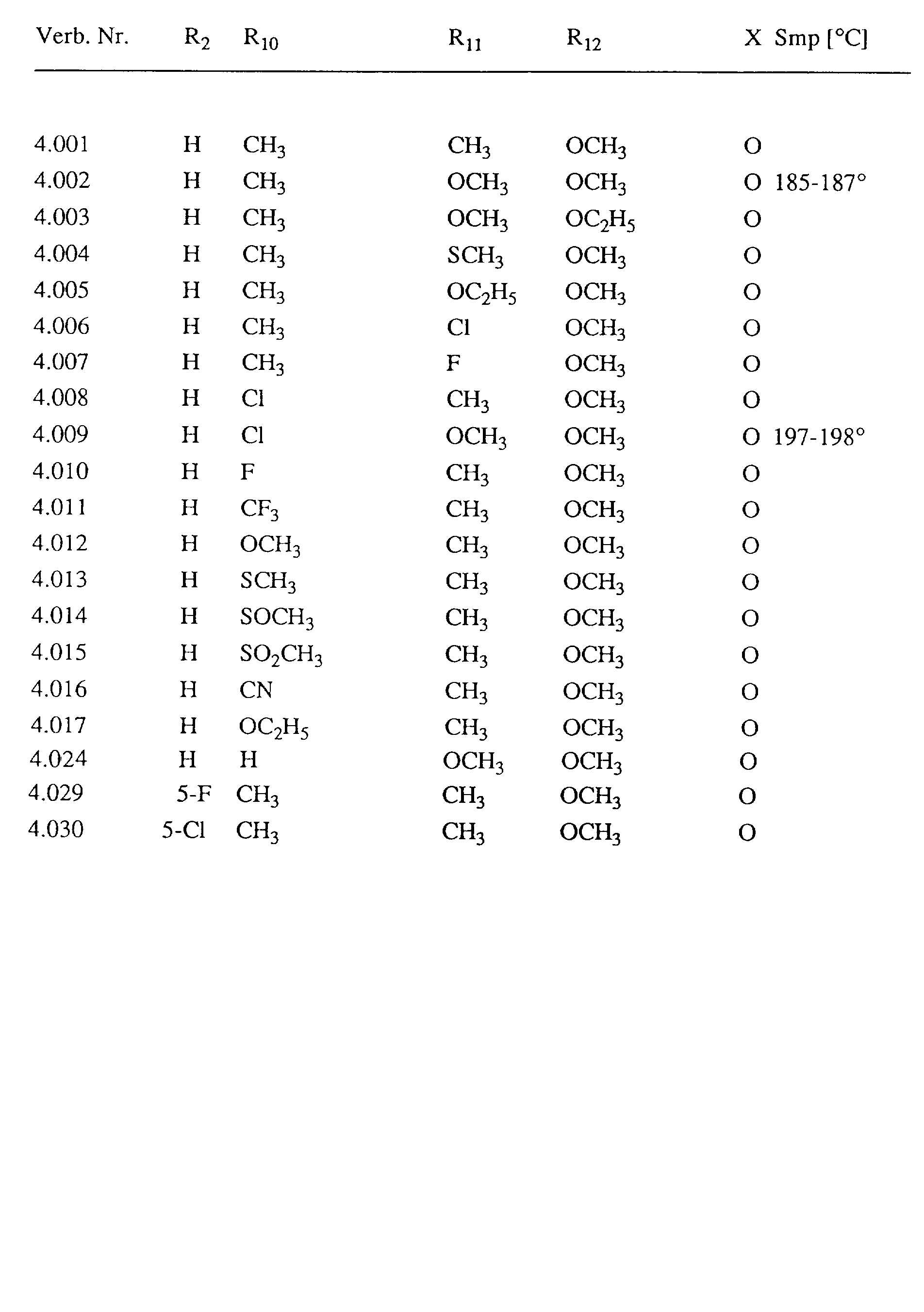

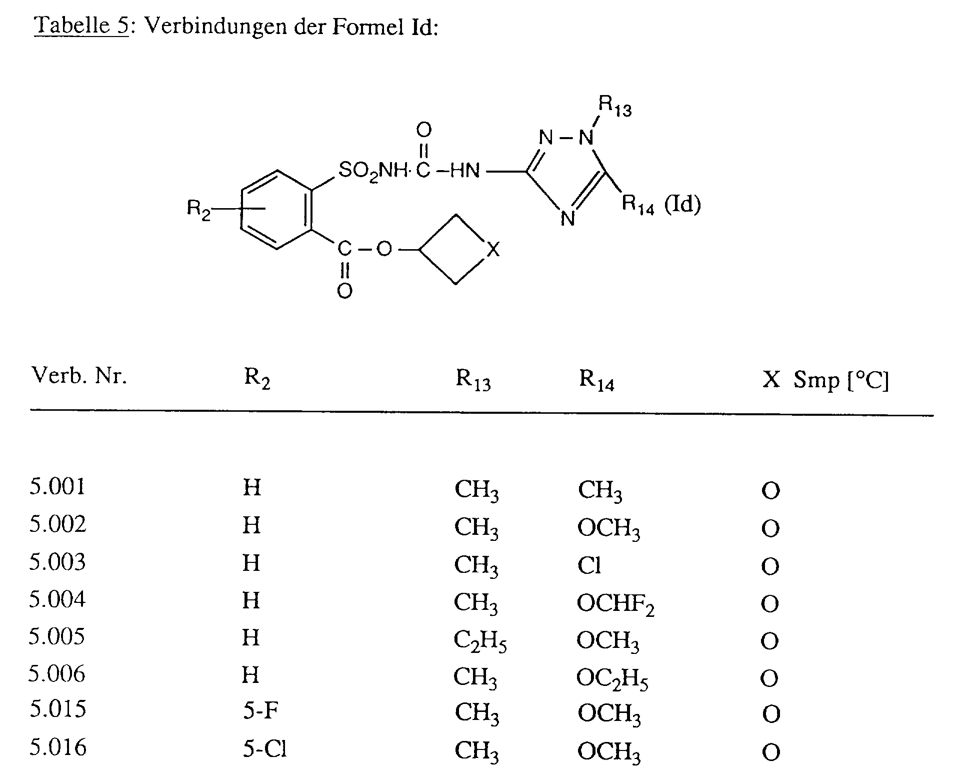

### Formulierungsbeispiele für Wirkstoffe der Formel I (% = Gewichtsprozent)

| 1. Spritzpulver | a) | b) | c) |
|---|---|---|---|
| Wirkstoff gemäß Tabellen 2-5 | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2 % | 2 |
| Hochdisperse Kieselsäure | 5 % | 27 % | 27 % |
| Kaolin | 67 % | - % | - |
| Natriumchlorid | - | - | 59,7 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsion-Konzentrate | a) | b) |
|---|---|---|
| Wirkstoff gemäß Tabellen 2-5 | 10% | 1 % |
| Ca-Dodecylbenzolsulfonat | 3 % | 3 % |
| Octylphenolpolyethylenglykolether (4-5 Mol AeO) | 3 % | 3 % |
| Ricinusöl-polyethylenglykolether (36 Mol AeO) | 4 % | 4 |
| Cyclohexanon | 30 | 10 % |
| Xylolgemisch | 50 % | 79 % |

Aus solchen Konzentraten können durch Verdünnen mit Wasser Emulsionen jeder gewünschten Konzentration hergestellt werden.

| 3. Stäubemittel | a) | b) |
|---|---|---|
| Wirkstoff gemäß Tabellen 2-5 | 0,1 % | 1 % |
| Talkum | 99,9 % | - |
| Kaolin | - | 99 % |

Durch inniges Vermischen der Trägerstoffe mit dem Wirkstoff erhält man gebrauchsfertige Stäubemittel.

| 4. Extruder-Granulat | a) | b) |
|---|---|---|
| Wirkstoff gemäß Tabellen 2-5 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschließend im Luftstrom getrocknet.

| 5. Umhüllungs-Granulat | |
|---|---|
| Wirkstoff gemäß Tabellen 2-5 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 6. Suspensions-Konzentrat | a) | b) |
|---|---|---|
| Wirkstoff gemäß Tabellen 2-5 | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 7. Salzlösung | |
|---|---|
| Wirkstoff gemäß Tabellen 2-5 | 5 % |
| Isopropylamin | 1 % |
| Octylphenolpolyethylenglycolether (78 Mol AeO) | 91 % |
| | |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

### Biologische Beispiele

### Beispiel B1: Herbizidwirkung vor dem Auflaufen der Pflanzen

Kunststofftöpfe werden mit expandiertem Vermiculit (Dichte: 0,135 g/cm³, Wasseradsorptionsvermögen: 0,565 1/1) gefüllt. Nach dem Sättigen des nicht adsorptiven Vermiculits mit einer wäßrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefäße werden anschließend in einer Klimakammer bei einer Temperatur von 20 °C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Gießwasser 0,5 % eines handelsüblichen Flüssigdüngers zugesetzt. 12 Tage nach der Aussaat wird der Versuch ausgewertet und die Wirkung auf die Versuchspflanzen nach dem folgenden Mäßstab bewertet:
1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle)

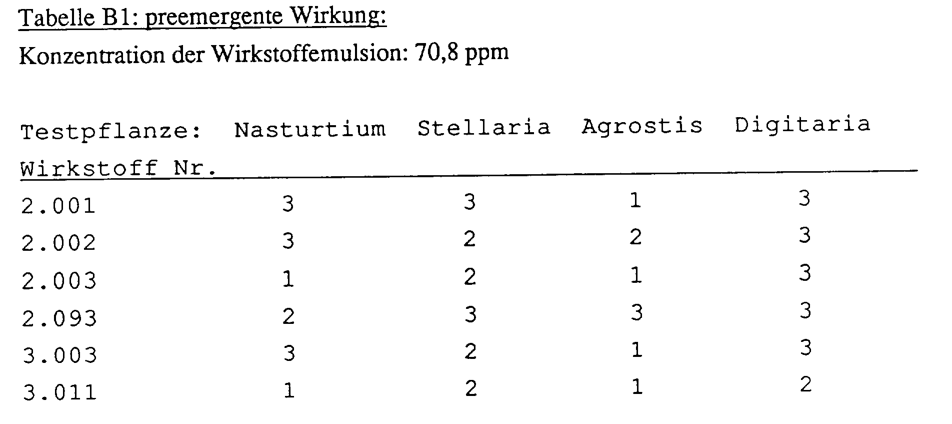

### Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wäßrigen Wirkstoffdispersion in einer Dosierung von 8-500 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet. Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Formel I starke Herbizidwirkung.

### Beispiel B3: Herbizidwirkung für Wasserreis (paddy)

Die Wasserunkräuter Echinochloa crus galli und Monocharia vag. werden in Plastikbechern (60 cm Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefäße. Die verwendete Dosis entspricht einer Wirkstoffmenge von 8-500 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Formel I schädigen dabei die Unkräuter.

## Patentansprüche (Patentansprüche für folgende(n) Vertragsstaat(en): AT, BE, CH, LI, DE, DK, FR, GB, IT, LU, NL, PT, SE)

1. Verbindungen Formel I 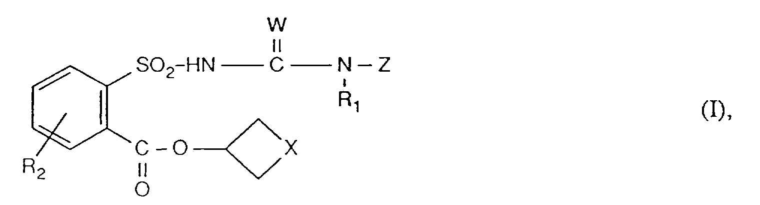 worin
X für Sauerstoff;
W für Sauerstoff oder Schwefel;
R₁ für Wasserstoff oder Methyl;
R₂ für Wasserstoff, Fluor, Chlor, Brom, Jod, (X)ₙR₃, NO₂, NR₄R₅, - C≡CR₆, 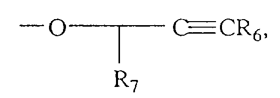 oder Cyano;
n für die Zahl 0 oder 1;
R₃ für C₁-C₄-Alkyl, oder durch 1-4 Halogenatome, C₁-C₃-Alkoxy oder C₁-C₃-Alkylthio substituiertes C₁-C₄-Alky-; C₂-C₄Alkenyl, oder durch 1-4 Halogenatome substituiertes C₂-C₄Alkenyl;
R₄ für Wasserstoff, CH₃O, CH₃CH₂O oder C₁-C₃-Alkyl;
R₅ für Wasserstoff oder C₁-C₃-Alkyl;
R₆ für Wasserstoff, Methyl oder Ethyl;
R₇ für Wasserstoff oder Methyl;
Z für 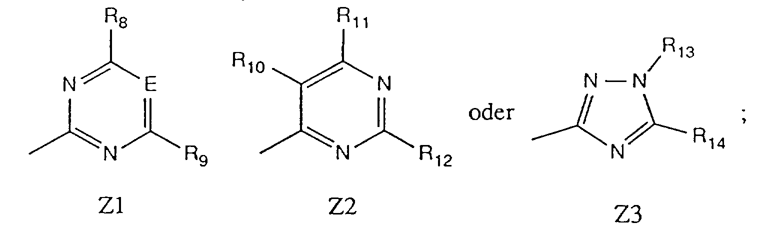
E für Methin oder Stickstoff;
R₈ für C₁-C₄-Alkyl, C₁-C₄-Alkoxy, C₁-C₄-Halogenalkoxy, C₁-C₄-Halogenalkyl, C₁-C₄-Halogenalkylthio, C₁-C₄-Alkylthio, Halogen, C₂-C₅-Alkoxyalkyl, C₂-C₅-Alkoxyalkoxy, Amino, C₁-C₃-Alkylamino oder Di-(C₁-C₃Alkyl)amino;
R₉ für C₁-C₄-Alkyl, C₁-C₄-Alkoxy, C₁-C₄-Halogenalkoxy, C₁-C₄-Halogenalkylthio, C₁-C₄-Alkylthio, C₂-C₅-Alkoxyalkyl, C₂-C₅-Alkoxyalkoxy, C₂-C₅-Alkylthioalkyl oder Cyclopropyl;
R₁₀ für Wasserstoff, Fluor, Chlor, Methyl, Trifluormethyl, CH₃O, CH₃CH₂O, CH₃S, CH₃SO, CH₃SO₂ oder Cyano;
R₁₁ für Methyl, Ethyl, CH₃O, CH₃CH₂O, Fluor oder Chlor;
R₁₂ für Methyl, Ethyl, CH₃O, CH₃CH₂O, Fluor oder Chlor;
R₁₃ für C₁-C₃-Alkyl;
R₁₄ für C₁-C₃-Alkyl, C₁-C₃-Alkoxy, Chlor oder OCHF₂ stehen;
sowie den Salzen dieser Verbindungen; mit den Maßgaben, daß
E für Methin steht wenn R₈ Halogen bedeutet; und
E für Methin steht wenn R₈ oder R₉ OCHF₂ oder SCHF₂ bedeuten.

2. Verbindungen der Formel I gemäß Anspruch 1, dadurch gekennzeichnet, daß W Sauerstoff bedeutet.

3. Verbindungen der Formel I gemäß Anspruch 2, dadurch gekennzeichnet, daß
Z für Z1; und
X für Sauerstoff steht.

4. Verbindungen der Formel I gemäß Anspruch 3, dadurch gekennzeichnet, daß E für Methin steht.

5. Verbindungen der Formel I gemäß Anspruch 3, dadurch gekennzeichnet, daß E fiir Stickstoff steht.

6. Verbindungen der Formel I gemäß einem der Ansprüche 4 und 5, dadurch gekennzeichnet, daß
R₂ für Wasserstoff, Fluor, Chlor, OCH₃, OCHF₂, Methyl, SCH₃, Methoxy, Ethoxy oder Chlorethoxy;
R₈ für C₁-C₃-ABky-, C₁-C₃-Alkoxy, C₁-C₂-Halogenalkoxy, Trifluormethyl, CHF₂, CH₂F, CH₂OCH₃, Fluor, Chlor, NH₂, NHCH₃, N(CH₃)₂, SCH₃ oder CH₂OCH₃; und
R₉ für C₁-C₃-Alky-, C₁-C₃-Alkoxy, C₁-C₂Halogenalkoxy oder Cyclopropyl stehen.

7. Verbindungen der Formel I gemäß Anspruch 6, dadurch gekennzeichnet, daß
R₂ für Wasserstoff;
R₈ für Methyl, Ethyl, OCH₃, OC₂H₅, OCHF₂, OCH₂CF₃, Chlor, NHCH₃, N(CH₃)₂ oder CH₂OCH₃; und
R₉ für Methyl, OCH₃, OCHF₂, OC₂H₅ oder Cyclopropyl stehen.

8. N-[2-(Oxetan-3-oxy-carbonyl)]-phenylsulfonyl-N'-(4-methoxy-6-methyl-1,3,5-triazinyl)-harnstoff gemäß Anspruch 5.

9. N-[2-(Oxetan-3-oxy-carbonyl)]-phenylsulfonyl-N'-(4,6-dimethyl-pyrimidin-2-yl)-harnstoff gemäß Anspruch 4.

10. N-[2-(Oxetan-3-oxy-carbonyl)]-phenylsulfonyl-N'-(4-methoxy-6-methyl-pyrimidin-2-yl)-harnstoff gemäß Anspruch 4.

11. N-[2-(Oxetan-3-oxy-carbonyl)]-phenylsulfonyl-N'-(4,6-dimethoxy-pyrimidin-2-yl)-harnstoff gemäß Anspruch 4.

12. Verfahren zur Herstellung der Verbindungen der Formel I, dadurch gekennzeichnet, daß man entweder
a) ein Phenylsulfonamid der Formel II 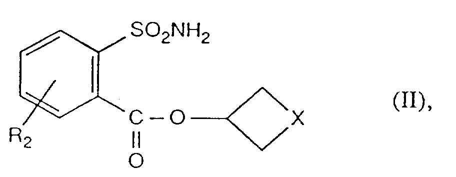 worin R₂ und X die unter Formel I in Anspruch 1 angegebene Bedeutung haben, in Gegenwart einer Base mit einem Pyrimidinyl-, Triazolyl- oder Triazinylcarbamat oder -thiocarbamat der Formel III 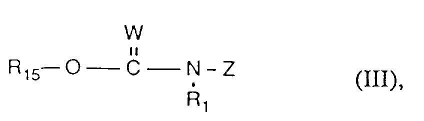 worin W, Z und R₁ die unter Formel I in Anspruch 1 angegebene Bedeutung haben und R₁₅ für Phenyl oder durch C₁-C₄-ABky- oder Halogen substituiertes Phenyl steht, umsetzt, oder
b) ein Sulfonylcarbamat oder -thiocarbamat der Formel IV 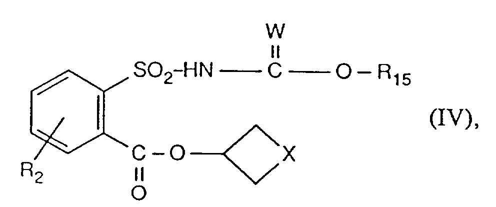 worin R₂, W, X und Z die unter Formel I in Anspruch 1 angegebene Bedeutung haben und R₁₅ die unter Formel III angegebene Bedeutung hat, in Gegenwart einer Base mit einem Amin der Formel V
H₂N-Z (V)
worin Z die unter Formel I in Anspruch 1 angegebene Bedeutung hat, umsetzt, oder
c) ein Phenylsulfonamid der Formel II 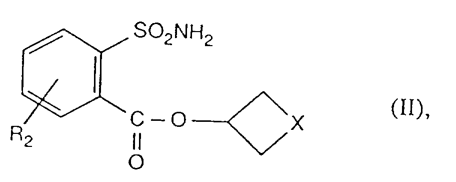 worin R₂ und X die unter Formel I in Anspruch 1 angegebene Bedeutung haben, in Gegenwart einer Base mit einem Pyrimidinyl-, Triazolyl- oder Triazinylisocyanat oder -isothiocyanat der Formel VII
Y=N=C-Z (VII)
worin Z die unter Formel I in Anspruch 1 angegebene Bedeutung hat und Y Sauerstoff oder Schwefel bedeutet, umsetzt.

13. Phenylsulfonamide der Formel II 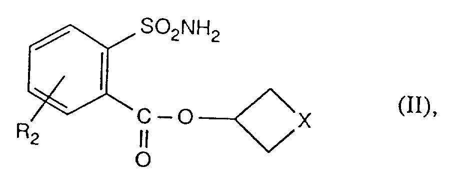 worin R₂ und X die unter Formel I in Anspruch 1 angegebene Bedeutung haben.

14. Phenylsulfochloride der Formel VIII 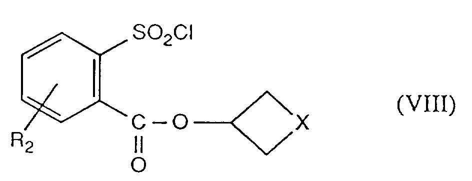 worin R₂ und X die unter Formel I in Anspruch 1 angegebene Bedeutung haben.

15. Sulfonylcarbamate oder -thiocarbamate der Formel IV 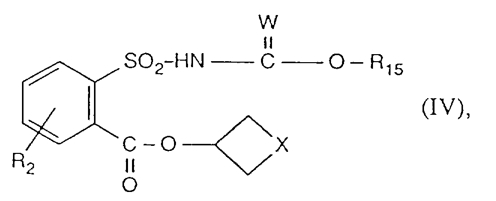 worin R₂, W, X und Z die unter Formel I in Anspruch 1 angegebene Bedeutung haben und R₁₅ für Phenyl oder durch C₁-C₄-Alkyl oder Halogen substituiertes Phenyl steht.

16. Ein herbizides und den Pflanzenwuchs hemmendes Mittel, gekennzeichnet durch einen Gehalt an einem oder mehreren Sulfonylharnstoffen und -thioharnstoffen der Formel I, gemäß Anspruch 1.

17. Mittel gemäß Anspruch 16, dadurch gekennzeichnet, daß es zwischen 0,1 % und 95 % Wirkstoff der Formel I gemäß Anspruch 1 enthält.

18. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

19. Verfahren gemäß Anspruch 18, dadurch gekennzeichnet, daß man eine Wirkstoffmenge zwischen 0,001 und 2 kg pro Hektar appliziert.

20. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

21. Verfahren gemäß Anspruch 18 zur selektiven pre- oder post-emergenten Bekämpfung von Unkräutern in Nutzpflanzenkulturen.

22. Verwendung eines Mittels gemäß Anspruch 16 zur selektiven pre- oder post-emergenten Bekämpfung von Unkräutern in Nutzpflanzenkulturen.

## Patentansprüche (Patentansprüche für folgende(n) Vertragsstaat(en): ES)

1. Verfahren zur Herstellung der Verbindungen der Formel I, 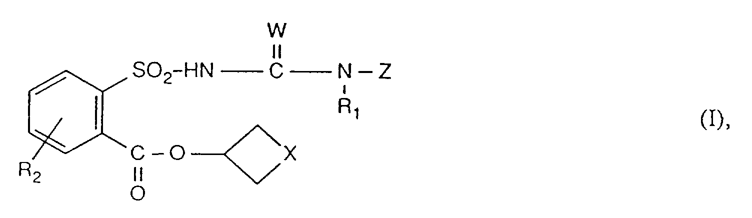 worin
X für Sauerstoff;
W für Sauerstoff oder Schwefel;
R₁ für Wasserstoff oder Methyl;
R₂ für Wasserstoff, Fluor, Chlor, Brom, Jod, (X)ₙR₃, NO₂, NR₄R₅, -C≡CR₆, 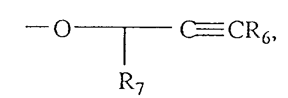 oder Cyano;
n für die Zahl 0 oder 1;
R₃ für C₁-C₄-Alkyl, oder durch 1-4 Halogenatome, C₁-C₃-Alkoxy oder C₁-C₃-Alkylthio substituiertes C₁-C₄-Alky-; C₂-C₄Alkenyl, oder durch 1-4 Halogenatome substituiertes C₂-C₄Alkenyl;
R₄ für Wasserstoff, CH₃O, CH₃CH₂O oder C₁-C₃-Alkyl;
R₅ für Wasserstoff oder C₁-C₃-Alky-;
R₆ für Wasserstoff, Methyl oder Ethyl;
R₇ für Wasserstoff oder Methyl;
Z für 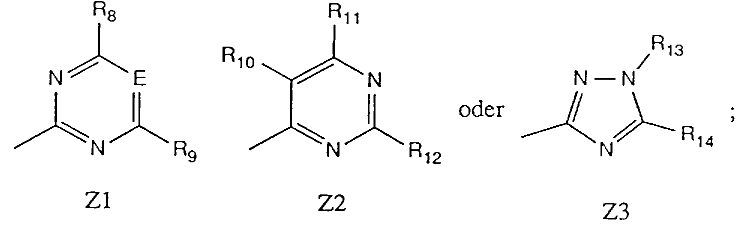
E für Methin oder Stickstoff;
R₈ für C₁-C₄-Alkyl, C₁-C₄-Alkoxy, C₁-C₄-Halogenalkoxy, C₁-C₄-Halogenalkyl, C₁-C₄-Halogenalkylthio, C₁-C₄-Alkythio, Halogen, C₂-C₅-Alkoxyalkyl, C₂-C₅-Alkoxyalkoxy, Amino, C₁-C₃-Alkylamino oder Di-(C₁-C₃Alkyl)amino;
R₉ für C₁-C₄-Alkyl, C₁-C₄-Alkoxy, C₁-C₄-Halogenalkoxy, C₁-C₄-Halogenalkylthio, C₁-C₄-Alkylthio, C₂-C₅-Alkoxyalkyl, C₂-C₅-Alkoxyalkoxy, C₂-C₅-Alkylthioalkyl oder Cyclopropyl;
R₁₀ für Wasserstoff, Fluor, Chlor, Methyl, Trifluormethyl, CH₃O, CH₃CH₂O, CH₃S, CH₃SO, CH₃SO₂ oder Cyano;
R₁₁ für Methyl, Ethyl, CH₃O, CH₃CH₂O, Fluor oder Chlor;
R₁₂ für Methyl, Ethyl, CH₃O, CH₃CH₂O, Fluor oder Chlor;
R₁₃ für C₁-C₃-Alkyl;
R₁₄ für C₁-C₃-Alkyl, C₁-C₃-Alkoxy, Chlor oder OCHF₂ stehen; sowie den Salzen dieser Verbindungen;
mit den Maßgaben, daß
E für Methin steht wenn R₈ Halogen bedeutet; und
E für Methin steht wenn R₈ oder R₉ OCHF₂ oder SCHF₂ bedeuten,
dadurch gekennzeichnet, daß man entweder
a) ein Phenylsulfonamid der Formel II 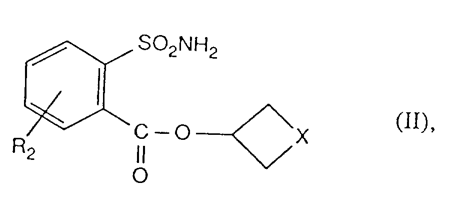 worin R₂ und X die unter Formel I angegebene Bedeutung haben, in Gegenwart einer Base mit einem Pyrimidinyl-, Triazolyl- oder Triazinylcarbamat oder -thiocarbamat der Formel III 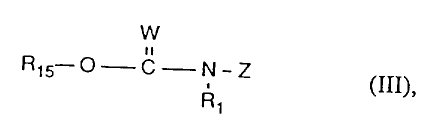 worin W, Z und R₁ die unter Formel I angegebene Bedeutung haben und R₁₅ für Phenyl oder durch C₁-C₄-Alkyl oder Halogen substituiertes Phenyl steht, umsetzt, oder
b) ein Sulfonylcarbamat oder -thiocarbamat der Formel IV 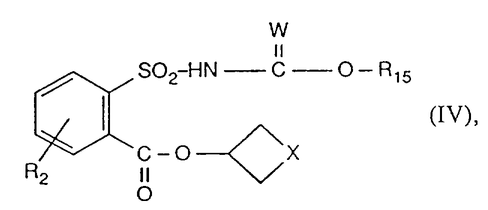 worin R₂, W, X und Z die unter Formel I angegebene Bedeutung haben und R₁₅ die unter Formel III angegebene Bedeutung hat, in Gegenwart einer Base mit einem Amin der Formel V
H₂N-Z (V)
worin Z die unter Formel I angegebene Bedeutung hat, umsetzt, oder
c) ein Phenylsulfonamid der Formel II 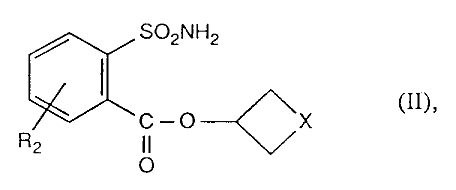 worin R₂ und X die unter Formel I angegebene Bedeutung haben, in Gegenwart einer Base mit einem Pyrimidinyl-, Triazolyl- oder Triazinylisocyanat oder -isothiocyanat der Formel VII
Y=N=C-Z (VII)
worin Z die unter Formel I angegebene Bedeutung hat und Y Sauerstoff oder Schwefel bedeutet, umsetzt.

2. Verfahren nach Anspruch 1 zur Herstellung von Verbindungen der Formel I, worin W Sauerstoff bedeutet.

3. Verfahren nach Anspruch 2 zur Herstellung von Verbindungen der Formel I, worin
Z für Z1; und
X für Sauerstoff steht.

4. Verfahren nach Anspruch 3 zur Herstellung von Verbindungen der Formel I, worin E für Methin steht.

5. Verfahren nach Anspruch 3 zur Herstellung von Verbindungen der Formel I, worin E für Stickstoff steht.

6. Verfahren nach Anspruch 4 und 5 zur Herstellung von Verbindungen der Formel I, worin
R₂ für Wasserstoff, Fluor, Chlor, OCH₃, OCHF₂, Methyl, SCH₃, Methoxy, Ethoxy oder Chlorethoxy;
R₈ für C₁-C₃-Alkyl, C₁-C₃-Alkoxy, C₁-C₂-Halogenalkoxy, Trifluormethyl, CHF₂, CH₂F, CH₂OCH₃, Fluor, Chlor, NH₂, NHCH₃, N(CH₃)₂, SCH₃ oder CH₂OCH₃; und
R₉ für C₁-C₃-Alkyl, C₁-C₃-Alkoxy, C₁-C₂Halogenalkoxy oder Cyclopropyl stehen.

7. Verfahren nach Anspruch 6 zur Herstellung von Verbindungen der Formel I, worin
R₂ für Wasserstoff;
R₈ für Methyl, Ethyl, OCH₃, OC₂H₅, OCHF₂, OCH₂CF₃, Chlor, NHCH₃, N(CH₃)₂ oder CH₂OCH₃; und
R₉ für Methyl, OCH₃, OCHF₂, OC₂H₅ oder Cyclopropyl stehen.

8. Verfahren nach Anspruch 5 zur Herstellung von N-[2-(Oxetan-3-oxy-carbonyl)]-phenylsulfonyl-N'-(4-methoxy-6-methyl-1,3,5-triazinyl)-harnstoff.

9. Verfahren nach Anspruch 4 zur Herstellung von N-[2-(Oxetan-3-oxy-carbonyl)]-phenylsulfonyl-N'-(4,6-dimethyl-pyrimidin-2-yl)-harnstoff.

10. Verfahren nach Anspruch 4 zur Herstellung von N-[2-(Oxetan-3-oxy-carbonyl)]phenylsulfonyl-N'-(4-methoxy-6-methyl-pyrimidin-2-yl)-harnstoff.

11. Verfahren nach Anspruch 4 zur Herstellung von N-[2-(Oxetan-3-oxy-carbonyl)]-phenylsulfonyl-N'-(4,6-dimethoxy-pyrimidin-2-yl)-harnstoff.

12. Ein herbizides und den Pflanzenwuchs hemmendes Mittel, gekennzeichnet durch einen Gehalt an einem oder mehreren Sulfonylharnstoffen und -thioharnstoffen der Formel I, gemäß Anspruch 1.

13. Mittel gemäß Anspruch 12, dadurch gekennzeichnet, daß es zwischen 0,1 % und 95 % Wirkstoff der Formel I gemäß Anspruch 1 enthält.

14. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

15. Verfahren gemäß Anspruch 14, dadurch gekennzeichnet, daß man eine Wirkstoffmenge zwischen 0,001 und 2 kg pro Hektar appliziert.

16. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

17. Verfahren gemäß Anspruch 14 zur selektiven pre- oder post-emergenten Bekämpfung von Unkräutern in Nutzpflanzenkulturen.

18. Verwendung eines Mittels gemäß Anspruch 12 zur selektiven pre- oder post-emergenten Bekämpfung von Unkräutern in Nutzpflanzenkulturen.

## Claims (Claims for the following Contracting State(s): AT, BE, CH, LI, DE, DK, FR, GB, GR, IT, LU, NL, PT, SE)

1. A compound of the formula I 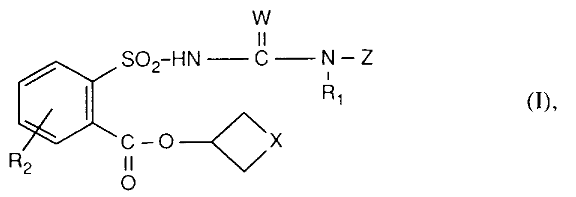 in which X is oxygen; W is oxygen or sulfur; R₁ is hydrogen or methyl; R₂ is hydrogen, fluorine, chlorine, bromine, iodine, (X)ₙR₃, NO₂, NR₄R₅, -C≡CR₆, 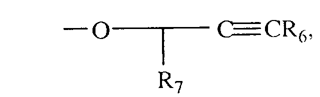 or cyano; n is the number 0 or 1; R₃ is C₁-C₄alkyl or C₁-C₄alkyl which is substituted by 1-4 halogen atoms, C₁-C₃alkoxy or C₁-C₃alkylthio; or C₂-C₄alkenyl or C₂-C₄alkenyl which is substituted by 1-4 halogen atoms; R₄ is hydrogen, CH₃O, CH₃CH₂O or C₁-C₃alkyl; R₅ is hydrogen or C₁-C₃alkyl; R₆ is hydrogen, methyl or ethyl; R₇ is hydrogen or methyl; Z is 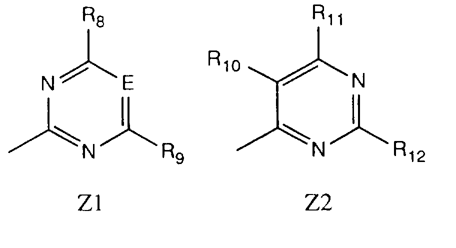 or 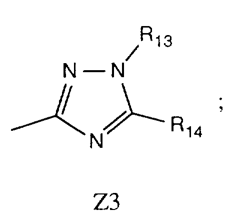 E is methine or nitrogen; R₈ is C₁-C₄alkyl, C₁-C₄alkoxy, C₁-C₄haloalkoxy, C₁-C₄haloalkyl, C₁-C₄haloalkylthio, C₁-C₄alkylthio, halogen, C₂-C₅alkoxyalkyl, C₂-C₅alkoxyalkoxy, amino, C₁-C₃alkylamino or di(C₁-C₃alkyl)amino; R₉ is C₁-C₄alkyl, C₁-C₄alkoxy, C₁-C₄haloalkoxy, C₁-C₄haloalkylthio, C₁-C₄alkylthio, C₂-C₅alkoxyalkyl, C₂-C₅alkoxyalkoxy, C₂-C₅alkylthioalkyl or cyclopropyl; R₁₀ is hydrogen, fluorine, chlorine, methyl, trifluoromethyl, CH₃O, CH₃CH₂O, CH₃S, CH₃SO, CH₃SO₂ or cyano; R₁₁ is methyl, ethyl, CH₃O, CH₃CH₂O, fluorine or chlorine; R₁₂ is methyl, ethyl, CH₃O, CH₃CH₂O, fluorine or chlorine; R₁₃ is C₁-C₃alkyl; and R₁₄ is C₁-C₃alkyl, C₁-C₃alkoxy, chlorine or OCHF₂; and the salts of this compound; with the provisos that E is methine if R₈ is halogen; and E is methine if R₈ or R₉ is OCHF₂ or SCHF₂.

2. A compound of the formula I according to claim 1, in which W is oxygen.

3. A compound of the formula I according to claim 2, in which Z is Z1; and X is oxygen.

4. A compound of the formula I according to claim 3, in which E is methine.

5. A compound of the formula I according to claim 3, in which E is nitrogen.

6. A compound of the formula I according to any one of claims 4 and 5, in which R₂ is hydrogen, fluorine, chlorine, OCH₃, OCHF₂, methyl, SCH₃, methoxy, ethoxy or chloroethoxy; R₈ is C₁-C₃alkyl, C₁-C₃alkoxy, C₁-C₂haloalkoxy, trifluoromethyl, CHF₂, CH₂F, CH₂OCH₃, fluorine, chlorine, NH₂, NHCH₃, N(CH₃)₂, SCH₃ or CH₂OCH₃; and R₉ is C₁-C₃alkyl, C₁-C₃alkoxy, C₁-C₂haloalkoxy or cyclopropyl.

7. A compound of the formula I according to claim 6, in which R₂ is hydrogen; R₈ is methyl, ethyl, OCH₃, OC₂H₅, OCHF₂, OCH₂CF₃, chlorine, NHCH₃, N(CH₃)₂ or CH₂OCH₃; and R₉ is methyl, OCH₃, OCHF₂, OC₂H₅ or cyclopropyl.

8. N-[2-(Oxetan-3-oxycarbonyl)](phenylsulfonyl-N'-(4-methoxy-6-methyl1,3,5-triazinyl)urea according to claim 5.

9. N-[2-(Oxetan-3-oxycarbonyl)]phenylsulfonyl-N'-(4,6-dimethylpyrimidin-2-yl)urea according to claim 4.

10. N-[2-(Oxetan-3-oxycarbonyl)]phenylsulfonyl-N'-(4-methoxy-6-methylpyrimidin-2-yl)urea according to claim 4.

11. N-[2-(Oxetan-3-oxycarbonyl)]phenylsulfonyl-N'-(4,6-dimethoxypyrimidin-2-yl) urea according to claim 4.

12. A process for the preparation of a compound of the formula I, which comprises
a) reacting a phenylsulfonamide of the formula II 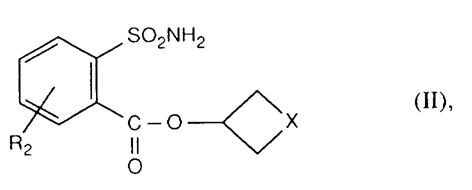 in which R₂ and X are as defined under formula I in claim 1, with a pyrimidinyl-, triazolyl- or triazinylcarbamate or -thiocarbamate of the formula III 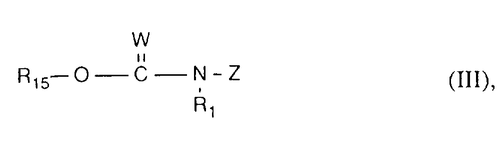 in which W, Z and R₁ are as defined under formula I in claim 1 and R₁₅ is phenyl or C₁-C₄alkyl- or halogen-substituted phenyl, in the presence of a base, or
b) reacting a sulfonylcarbamate or -thiocarbamate of the formula IV 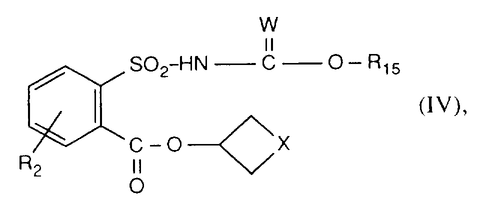 in which R₂, W, X and Z are as defined under formula I in claim 1 and R₁₅ is as defined under formula III, with an amine of the formula V
H₂N-Z (V)
in which Z is as defined under formula I in claim 1, in the presence of a base, or
c) reacting a phenylsulfonamide of the formula II 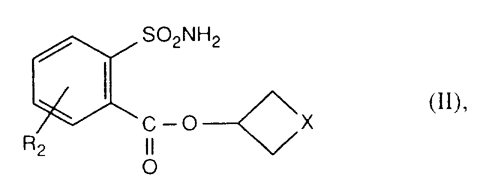 in which R₂ and X are as defined under formula I in claim 1, with a pyrimidinyl-, triazolyl- or triazinyl isocyanate or isothiocyanate of the formula VII
Y=N=C-Z (VII)
in which Z is as defined under formula I in claim 1 and Y is oxygen or sulfur, in the presence of a base.

13. A phenylsulfonamide of the formula II 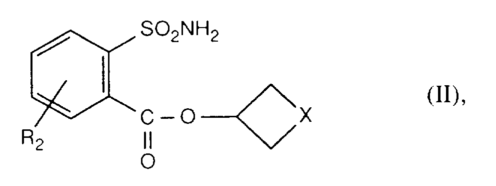 in which R₂ and X are as defined under formula I in claim 1.

14. A phenylsulfonyl chloride of the formula VIII 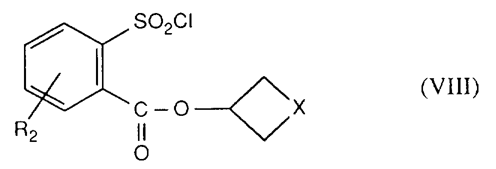 in which R₂ and X are as defined under formula I in claim 1.

15. A sulfonylcarbamate or -thiocarbamate of the formula IV 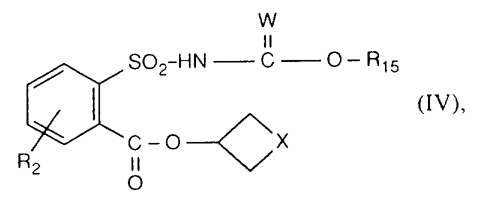 in which R₂, W, X and Z are as defined under formula I in claim 1 and R₁₅ is phenyl or phenyl which is substituted by C₁-C₄alkyl or halogen.

16. A herbicidal and plant growth-inhibiting composition which contains one or more sulfonylureas and -thioureas of the formula I according to claim 1.

17. A composition according to claim 19, which contains between 0.1% and 95 % of the active ingredient of the formula I according to claim 1.

18. A method for control of undesirable plant growth, which comprises applying an effective amount of an active ingredient of the formula I according to claim 1 or a composition containing this active ingredient to the plants or their environment.

19. The method according to claim 18, wherein an amount of active ingredient of between 0.001 and 2 kg per hectare is applied.

20. A method of inhibiting plant growth, which comprises applying an effective amount of an active ingredient of the formula I according to claim 1 or a composition containing this active ingredient to the plants or their environment.

21. The method according to claim 18 for selective pre- or postemergence control of weeds in crops of useful plants.

22. The use of a composition according to claim 16 for selective pre- or postemergence control of weeds in crops of useful plants.

## Claims (Claims for the following Contracting State(s): ES)

1. A process for the preparation of a compound of the formula I 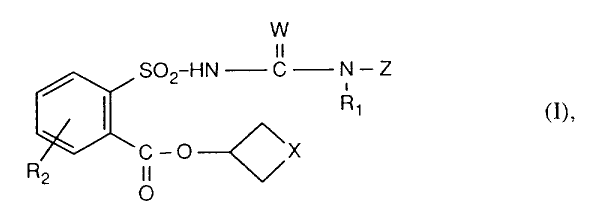 in which X is oxygen; W is oxygen or sulfur; R₁ is hydrogen or methyl; R₂ is hydrogen, fluorine, chlorine, bromine, iodine, (X)ₙR₃, NO₂, NR₄R₅, -C≡CR₆, 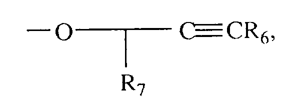 or cyano; n is the number 0 or 1; R₃ is C₁-C₄alkyl or C₁-C₄alkyl which is substituted by 1-4 halogen atoms, C₁-C₃alkoxy or C₁-C₃alkylthio; or C₂-C₄alkenyl or C₂-C₄alkenyl which is substituted by 1-4 halogen atoms; R₄ is hydrogen, CH₃O, CH₁CH₃O or C₁-C₃alkyl; R₅ is hydrogen or C₁-C₃alkyl; R₆ is hydrogen, methyl or ethyl; R₇ is hydrogen or methyl; Z is 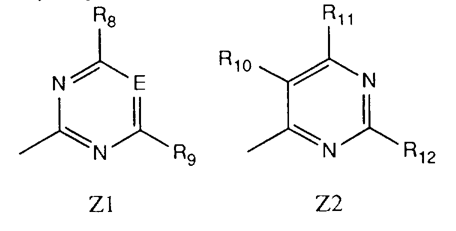 or 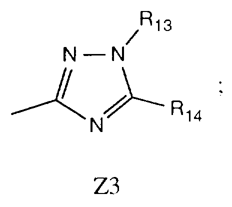 E is methine or nitrogen; R₈ is C₁-C₄alkyl, C₁-C₄alkoxy, C₁-C₄haloalkoxy, C₁-C₄haloalkyl, C₁-C₄haloalkylthio, C₁-C₄alkylthio, halogen, C₂-C₅alkoxyalkyl, C₂-C₅alkoxyalkoxy, amino, C₁-C₃alkylamino or di(C₁-C₃alkyl)amino; R₉ is C₁-C₄alkyl, C₁-C₄alkoxy, C₁-C₄haloalkoxy, C₁-C₄haloalkylthio, C₁-C₄alkylthio, C₂-C₅alkoxyalkyl, C₂-C₅alkoxyalkoxy, C₂-C₅alkylthioalkyl or cyclopropyl; R₁₀ is hydrogen, fluorine, chlorine, methyl, trifluoromethyl, CH₃O, CH₃CH₂O, CH₃S, CH₃SO, CH₃SO₂ or cyano; R₁₁ is methyl, ethyl, CH₃O, CH₃CH₂O, fluorine or chlorine; R₁₂ is methyl, ethyl, CH₃O, CH₃CH₂O, fluorine or chlorine; R₁₃ is C₁-C₃alkyl; and R₁₄ is C₁-C₃alkyl, C₁-C₃alkoxy, chlorine or OCHF₂; and the salts of this compound; with the provisos that E is methine if R₈ is halogen; and E is methine if R₈ or R₉ is OCHF₂ or SCHF₂, which comprises
a) reacting a phenylsulfonamide of the formula II 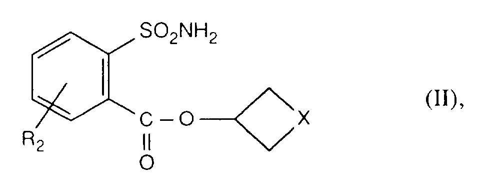 in which R₂ and X are as defined under formula I, with a pyrimidinyl-, triazolyl- or triazinylcarbamate or -thiocarbamate of the formula III 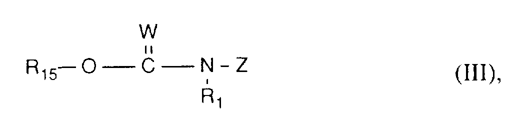 in which W, Z and R₁ are as defined under formula I and R₁₅ is phenyl or C₁ -C₄alkyl- or halogen-substituted phenyl, in the presence of a base, or
b) reacting a sulfonylcarbamate or -thiocarbamate of the formula IV 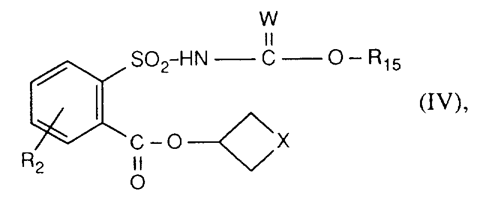 in which R₂, W, X and Z are as defined under formula I and R₁₅ is as defined under formula III, with an amine of the formula V
H₂N-Z (V)
in which Z is as defined under formula I, in the presence of a base, or
c) reacting a phenylsulfonamide of the formula II 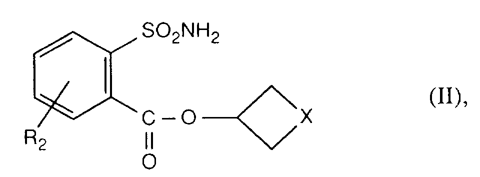 in which R₂ and X are as defined under formula I, with a pyrimidinyl-, triazolyl- or triazinyl isocyanate or isothiocyanate of the formula VII
Y=N=C-Z (VII)
in which Z is as defined under formula I and Y is oxygen or sulfur, in the presence of a base.

2. A process according to claim 1 for the preparation of a compound of the formula I in which W is oxygen.

3. A process according to claim 2 for the preparation of a compound of the formula I in which Z is Z1 ; and X is oxygen.

4. A process according to claim 3 for the preparation of a compound of the formula I in which E is methine.

5. A process according to claim 3 for the preparation of a compound of the formula I in which E is nitrogen.

6. A process according to claim 4 and 5 for the preparation of a compound of the formula I in which R₂ is hydrogen, fluorine, chlorine, OCH₃, OCHF₂, methyl, SCH₃, methoxy, ethoxy or chloroethoxy; R₈ is C₁-C₃alkyl, C₁-C₃alkoxy, C₁-C₂haloalkoxy, trifluoromethyl, CHF₂, CH₂F, CH₂OCH₃, fluorine, chlorine, NH₂, NHCH₃, N(CH₃)₂, SCH₃ or CH₂OCH₃; and R₉ is C₁-C₃alkyl, C₁-C₃alkoxy, C₁-C₂haloalkoxy or cyclopropyl.

7. A process according to claim 6 for the preparation of a compound of the formula I in which R₂ is hydrogen; R₈ is methyl, ethyl, OCH₃, OC₂H₅, OCHF₂, OCH₂CF₃, chlorine, NHCH₃, N(CH₃)₂ or CH₂OCH₃; and R₉ is methyl, OCH₃, OCHF₂, OC₂H₅ or cyclopropyl.

8. A process according to claim 5 for the preparation of N-[2-(oxetan-3-oxycarbonyl)]phenylsulfonyl-N'-(4-methoxy-6-methyl-1,3,5-triazinyl)-urea.

9. A process according to claim 4 for the preparation of N-[2-(oxetan-3-oxycarbonyl)]phenylsulfonyl-N'-(4,6-dimethylpyrimidin-2-yl)urea.

10. A process according to claim 4 for the preparation of N-[2-(oxetan-3-oxycarbonyl)]phenylsulfonyl-N'-(4-methoxy-6-methylpyrimidin-2-yl)urea.

11. A process according to claim 4 for the preparation of N-[2-(oxetan-3-oxycarbonyl)]phenylsulfonyl-N'-(4,6-dimethoxypyrimidin-2-yl)urea.

12. A herbicidal and plant growth-inhibiting composition which contains one or more sulfonylureas and -thioureas of the formula I according to claim 1.

13. A composition according to claim 12, which contains between 0.1% and 95 % of the active ingredient of the formula I according to claim 1.

14. A method for control of undesirable plant growth, which comprises applying an effective amount of an active ingredient of the formula I according to claim 1 or a composition containing this active ingredient to the plants or their environment.

15. The method according to claim 14, wherein an amount of active ingredient of between 0.001 and 2 kg per hectare is applied.

16. A method of inhibiting plant growth, which comprises applying an effective amount of an active ingredient of the formula I according to claim 1 or a composition containing this active ingredient to the plants or their environment.

17. The method according to claim 14 for selective pre- or postemergence control of weeds in crops of useful plants.

18. The use of a composition according to claim 12 for selective pre- or postemergence control of weeds in crops of useful plants.

## Revendications (Revendications pour l'(les) Etat(s) contractant(s) suivant(s): AT, BE, CH, LI, DE, DK, FR, GB, GR, IT, LU, NL, PT, SE)

1. Composés de formule I 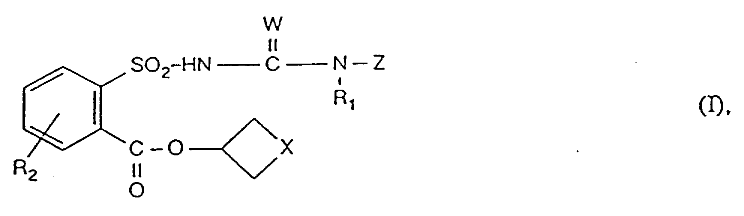 où
X est un oxygène ;
W est un oxygène ou un soufre ;
R₁ est un hydrogène ou un méthyle ;
R₂ est un hydrogène, fluor, chlore, brome, iode, (X)ₙR₃, NO₂, NR₄R_{5,}-C≡CR₆ 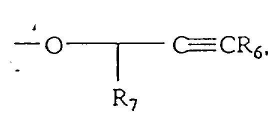 ou cyano ;
n est le nombre 0 ou 1 ;
R₃ est un alkyle C₁₋₄, ou un alkyle C₁₋₄ substitué par 1-4 atomes d'halogène, un alcoxy C₁₋₃ ou alkylthio C₁₋₃ ; alcényle C₂₋₄ ou un alcényle C₂₋₄ substitué par 1-4 atomes d'halogène ;
R₄ est un hydrogène, CH₃O, CH₃CH₂O ou alkyle C₁₋₃ ;
R₅ est un hydrogène ou alkyle C₁₋₃ ;
R₆ est un hydrogène, méthyle ou éthyle ;
R₇ est un hydrogène ou méthyle ;
Z est 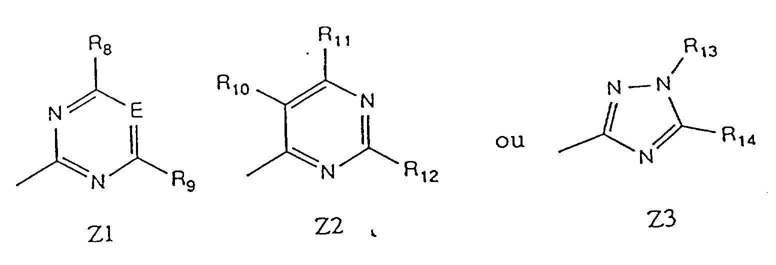
E est un méthine ou azote ;
R₈ est un alkyle C₁₋₄, alcoxy C₁₋₄, halogénoalcoxy C₁₋₄, halogénoalkyle C₁₋₄, halogénoalkylthio C₁₋₄, alkylthio C₁₋₄, halogène, alcoxyalkyle C₂₋₅, alcoxyalcoxy C₂₋₅, amino, alkylamino C₁₋₃ ou di-(alkyle C₁₋₃)amino ;
R₉ est un alkyle C₁₋₄, alcoxy C₁₋₄, halogénoalcoxy C₁₋₄, halogénoalkylthio C₁₋₄, alkylthio C₁₋₄, alcoxyalkyle C₂₋₅, alcoxyalcoxy C₂₋₅, alkylthioalkyle C₂₋₅ ou cyclopropyle ;
R₁₀ est un hydrogène, fluor, chlore, méthyle, trifluorométhyle, CH₃O, CH₃CH₂O, CH₃S, CH₃SO, CH₃SO₂ ou cyano ;
R₁₁ est un méthyle, éthyle, CH₃O, CH₃CH₂O, fluor ou chlore ;
R₁₂ est un méthyle, éthyle, CH₃O, CH₃CH₂O, fluor ou chlore ;
R₁₃ est un alkyle C₁₋₃ ;
R₁₄ est un alkyle C₁₋₃, alcoxy C₁₋₃, chlore ou OCHF₂ ;
ainsi que les sels de ces composés ;
à la condition que
E représente le méthine quand R₈ représente un halogène ; et
E représente le méthine quand R₈ ou R₉ représente OCHF₂ ou SCHF₂.

2. Composés de formule I selon la revendication 1, caractérisés en ce que W représente l'oxygène.

3. Composés de formule I selon la revendication 2, caractérisés en ce que Z représente Z1 ; et
X représente l'oxygène.

4. Composés de formule I selon la revendication 3, caractérisés en ce que E représente le méthine.

5. Composés de formule I selon la revendication 3, caractérisés en ce que E représente l'azote.

6. Composés de formule I selon l'une des revendications 4 et 5, caractérisés en ce que
R₂ représente un hydrogène, fluor, chlore, OCH₃, OCHF₂, méthyle, SCH₃, méthoxy, éthoxy ou chloroéthoxy ;
R₈ est un alkyle C₁₋₃, alcoxy C₁₋₃, halogénoalcoxy C₁₋₂, trifluorométhyle, CHF₂, CH₂F, CH₂OCH₃, fluor, chlore, NH₂, NHCH₃, N(CH₃)₂, SCH₃ ou CH₂OCH₃ ; et
R₉ est un alkyle C₁₋₃, alcoxy C₁₋₃, halogénoalcoxy C₁₋₂ ou cyclopropyle.

7. Composés de formule I selon la revendication 6, caractérisés en ce que R₂ est un hydrogène ;
R₈ est un méthyle, éthyle, OCH₃, OC₂H₅, OCHF₂, OCH₂CF₃, chlore, NHCH₃, N(CH₃)₂ ou CH₂OCH₃ ; et
R₉ est un méthyle, OCH₃, OCHF₂, OC₂H₅, ou un cyclopropyle.

8. N-[2-(oxétan-3-oxy-carbonyl)]phénylsulfonyl-N'-(4-méthoxy-6-méthyl-1,3,5-triazinyl)-urée selon la revendication 5.

9. N-[2-(oxétan-3-oxy-carbonyl)]-phénylsulfonyl-N'-(4,6-diméthyl-pyrimidin-2-yl)-urée selon la revendication 4.

10. N-[2-(oxétan-3-oxy-carbonyl)]-phénylsulfonyl-N'-(4-méthoxy-6-méthyl-pyrimidin-2-yl) -urée selon la revendication 4.

11. N-[2-(oxétan-3-oxycarbonyl)]-phénylsulfonyl-N'-(4,6-diméthoxy-pyrimidin-2-yl)-urée selon la revendication 4.

12. Procédé de préparation de composés de formule I, caractérisé en ce que
a) on fait réagir un phénylsulfonamide de formule II 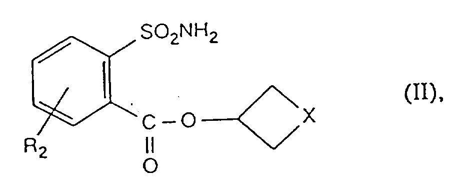 où R₂ et X ont la signification donnée à la revendication 1 pour la formule I, en présence d'une base avec un pyrimidinyl-, triazolyl- ou triazinyl- carbamate ou -thiocarbamate de formule III 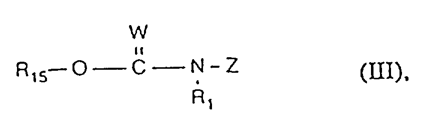 où W, Z et R₁ ont la signification donnée pour la formule I à la revendication 1, et R₁₅ représente un phényle ou phényle substitué par alkyle C₁₋₄ ou halogène, soit
b) on fait réagir un sulfonylcarbamate ou - thiocarbamate de formule IV 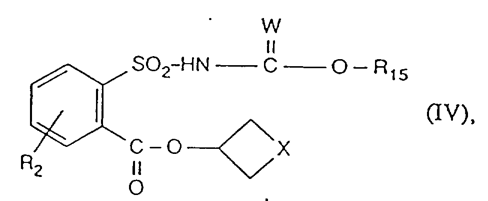 où R₂, W, X et Z ont la signification donnée pour la formule I à la revendication 1, et R₁₅ a la signification donnée à la formule III, en présence d'une base avec une amine de formule V
H₂N-Z (V)
où Z a la signification donnée pour la formule I à la revendication 1, soit
c) on fait réagir un phénylsulfonamide de formule II 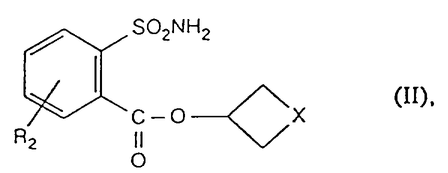 où R₂ et X ont la signification donnée pour la formule I à la revendication 1, en présence d'une base avec un pyrimidinyl-, triazolyl- ou triazinylisocyanate ou -isothiocyanate de formule VII
Y=N=C-Z (VII)
où Z a la signification donnée pour la formule I à la revendication 1, et Y représente un oxygène ou un soufre.

13. Phénylsulfonamides de formule II 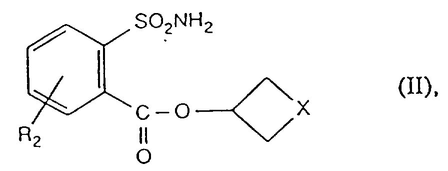 où R₂ et X ont la signification donnée pour la formule I à la revendication 1.

14. Phénylsulfochlorures de formule VIII 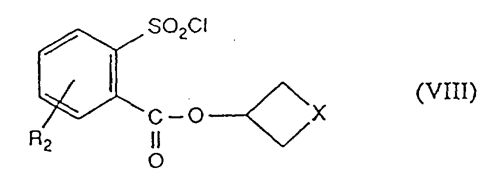 où R₂ et X ont la signification donnée pour la formule I à la revendication 1.

15. Sulfonylcarbamates ou -thiocarbamates de formule IV 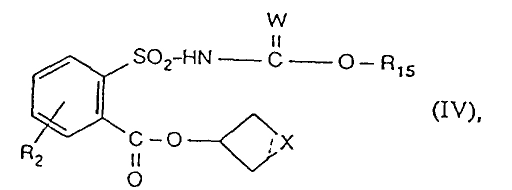 où R₂, W, X et Z ont la signification donnée pour la formule I à la revendication 1 et R₁₅ représente un phényle ou un phényle substitué par un halogène ou alkyle C₁₋₄.

16. Moyen inhibant la croissance des plantes et herbicide, caractérisé par une teneur en une ou plusieurs sulfonylurées et -thiourées de formule I selon la revendication 1.

17. Moyen selon la revendication 16, caractérisé en ce qu'il contient entre 0,1% et 95% de principe actif de formule I selon la revendication 1.

18. Procédé de lutte contre la croissance de plantes indésirables, caractérisé en ce qu'on applique un principe actif de formule I, selon la revendication 1, ou un moyen qui contient ce principe actif, en une quantité efficace sur les plantes ou sur leur espace vital.

19. Procédé selon la revendication 18, caractérisé en ce qu'on applique une quantité de principe actif entre 0,001 et 2 kg par hectare.

20. Procédé d'inhibition de la croissance de plantes, caractérisé en ce qu'on applique un principe actif de formule I, selon la revendication 1, ou un moyen contenant ce principe actif, en une quantité efficace sur les plantes ou leur espace vital.

21. Procédé selon la revendication 18, pour combattre sélectivement de façon pré- ou post-émergente des mauvaises herbes présentes dans les cultures de plantes utiles.

22. Utilisation d'un moyen selon la revendication 16 pour combattre sélectivement de façon pré- ou post-émergente des mauvaises herbes présentes dans les cultures de plantes utiles.

## Revendications (Revendications pour l'(les) Etat(s) contractant(s) suivant(s): ES)

1. Procédé de préparation de composés de formule I, 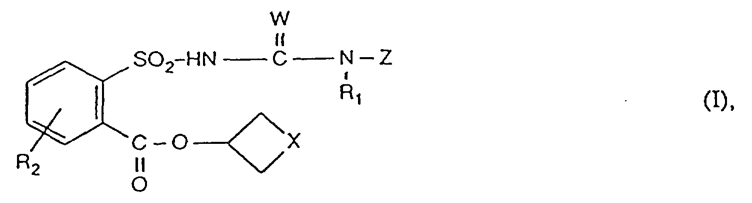 où
X est un oxygène ;
W est un oxygène ou un soufre ;
R₁ est un hydrogène ou un méthyle ;
R₂ est un hydrogène, fluor, chlore, brome, iode, (X)ₙR₃, NO₂, NR₄R₅,-C≡CR₆ 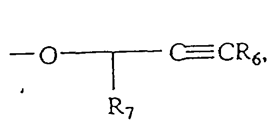 ou cyano ;
n est le nombre 0 ou 1 ;
R₃ est un alkyle C₁₋₄, ou un alkyle C₁₋₄ substitué par 1-4 atomes d'halogène, un alcoxy C₁₋₃ ou alkylthio C₁₋₃ ; alcényle C₂₋₄ ou un alcényle C₂₋₄ substitué par 1-4 atomes d'halogène ;
R₄ est un hydrogène, CH₃O, CH₃CH₂O ou alkyle C₁₋₃ ;
R₅ est un hydrogène ou alkyle C₁₋₃ ;
R₆ est un hydrogène, méthyle ou éthyle ;
R₇ est un hydrogène ou méthyle ;
Z est 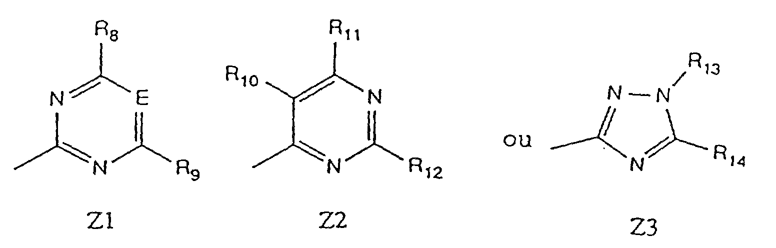
E est un méthine ou azote ;
R₈ est un alkyle C₁₋₄, alcoxy C₁₋₄, halogénoalcoxy C₁₋₄, halogénoalkyle C₁₋₄, halogénoalkylthio C₁₋₄, alkylthio C₁₋₄, halogène, alcoxyalkyle C₂₋₅, alcoxyalcoxy C₂₋₅, amino, alkylamino C₁₋₃ ou di-(alkyle C₁₋₃)amino ;
R₉ est un alkyle C₁₋₄, alcoxy C₁₋₄, halogénoalcoxy C₁₋₄, halogénoalkylthio C₁₋₄, alkylthio C₁₋₄, alcoxyalkyle C₂₋₅, alcoxyalcoxy C₂₋₅, alkylthioalkyle C₂₋₅ ou cyclopropyle ;
R₁₀ est un hydrogène, fluor, chlore, méthyle, trifluorométhyle, CH₃O, CH₃CH₂O, CH₃S, CH₃SO, CH₃SO₂ ou cyano ;
R₁₁ est un méthyle, éthyle, CH₃O, CH₃CH₂O, fluor ou chlore ;
R₁₂ est un méthyle, éthyle, CH₃O, CH₃CH₂O, fluor ou chlore ;
R₁₃ est un alkyle C₁₋₃ ;
R₁₄ est un alkyle C₁₋₃, alcoxy C₁₋₃, chlore ou OCHF₂ ;
ainsi que les sels de ces composés ;
à la condition que
E représente le méthine quand R₈ représente un halogène ; et
E représente le méthine quand R₈ ou R₉ représente OCHF₂ ou SCHF₂,
caractérisé en ce que
a) on fait réagir un phénylsulfonamide de formule II 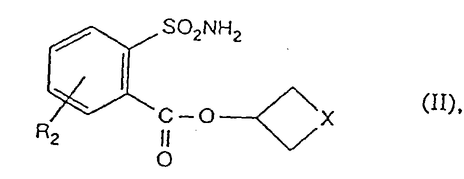 où R₂ et X ont la signification donnée à la revendication 1 pour la formule I, en présence d'une base avec un pyrimidinyl-, triazolyl- ou triazinyl- carbamate ou -thiocarbamate de formule III 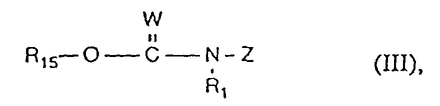 où W, Z et R₁ ont la signification donnée pour la formule I à la revendication 1, et R15 représente un phényle ou phényle substitué par alkyle C₁₋₄ ou halogène, soit
b) on fait réagir un sulfonylcarbamate ou - thiocarbamate de formule IV 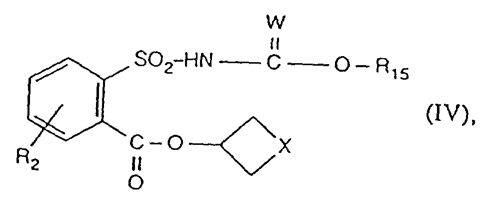 où R₂, W, X et Z ont la signification donnée pour la formule I à la revendication 1, et R₁₅ a la signification donnée à la formule III, en présence d'une base avec une amine de formule V
H₂N-Z (V)
où Z a la signification donnée pour la formule I à la revendication 1, soit
c) on fait réagir un phénylsulfonamide de formule II 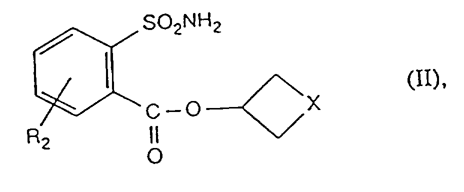 où R₂ et X ont la signification donnée pour la formule I à la revendication 1, en présence d'une base avec un pyrimidinyl-, triazolyl- ou triazinylisocyanate ou -isothiocyanate de formule VII
Y=N=C-Z (VII)
où Z a la signification donnée pour la formule I à la revendication 1, et Y représente un oxygène ou un soufre.

2. Procédé selon la revendication 1 de préparation de composés de formule I, où W représente l'oxygène.

3. Procédé selon la revendication 2 pour préparer des composés de formule I, où Z représente Z1 ; et
X représente l'oxygène.

4. Procédé selon la revendication 3 pour préparer des composés de formule I, où E est le méthine.

5. Procédé selon la revendication 3 pour préparer des composés de formule I, où E est l'azote.

6. Procédé selon la revendication 4 et 5 pour préparer des composés de formule I, où
R₂ représente un hydrogène, fluor, chlore, OCH₃, OCHF₂, méthyle, SCH₃, méthoxy, éthoxy ou chloroéthoxy ;
R₈ est un alkyle C₁₋₃, alcoxy C₁₋₃, halogénoalcoxy C₁₋₂, trifluorométhyle, CHF₂, CH₂F, CH₂OCH₃, fluor, chlore, NH₂, NHCH₃, N(CH₃)₂, SCH₃ ou CH₂OCH₃ ; et
R₉ est un alkyle C₁₋₃, alcoxy C₁₋₃, halogénoalcoxy C₁₋₂ ou cyclopropyle.

7. Procédé selon la revendication 6 pour préparer des composés de formule I où
R₂ est un hydrogène ;
R₈ est un méthyle, éthyle, OCH₃, OC₂H₅, OCHF₂, OCH₂CF₃, chlore, NHCH₃, N(CH₃)₂ ou CH₂OCH₃ ; et
R₉ est un méthyle, OCH₃, OCHF₂, OC₂H₅, ou un cyclopropyle.

8. Procédé selon la revendication 5 pour préparer la N-[2-(oxétan-3-oxy-carbonyl)]phénylsulfonyl-N'-(4-méthoxy-6-méthyl-1,3,5-triazinyl)-urée.

9. Procédé selon la revendication 4 pour préparer la N- [2-(oxétan-3-oxy-carbonyl)]-phénylsulfonyl-N'-(4,6-diméthyl-pyrimidin-2-yl)-urée.

10. Procédé selon la revendication 4 pour préparer la N-[2-(oxétan-3-oxy-carbonyl)]-phénylsulfonyl-N'-(4-méthoxy-6-méthyl-pyrimidin-2-yl)-urée.

11. Procédé selon la revendication 4 pour préparer la N-[2-(oxétan-3-oxycarbonyl)]-phénylsulfonyl-N'-(4,6-diméthoxy-pyrimidin-2-yl)-urée.

12. Moyen inhibant la croissance des plantes et herbicide, caractérisé par une teneur en une ou plusieurs sulfonylurées et -thiourées de formule I selon la revendication 1.

13. Moyen selon la revendication 12, caractérisé en ce qu'il contient entre 0,1% et 95% de principe actif de formule I selon la revendication 1.

14. Procédé de lutte contre la croissance de plantes indésirables, caractérisé en ce qu'on applique un principe actif de formule I, selon la revendication 1, ou un moyen qui contient ce principe actif, en une quantité efficace sur les plantes ou sur leur espace vital.

15. Procédé selon la revendication 14, caractérisé en ce qu'on applique une quantité de principe actif comprise entre 0,001 et 2 kg par hectare.

16. Procédé d'inhibition de la croissance de plantes, caractérisé en ce qu'on applique un principe actif de formule I, selon la revendication 1, ou un moyen contenant ce principe actif, en une quantité efficace sur les plantes ou leur espace vital.

17. Procédé selon la revendication 14, pour combattre sélectivement de manière pré- ou post-émergente les mauvaises herbes présentes dans les cultures de plantes utiles.

18. Utilisation d'un moyen selon la revendication 12 pour combattre sélectivement de manière pré- ou post-émergente les mauvaises herbes présentes dans les cultures de plantes utiles.
